# EUROPEAN PATENT APPLICATION

(11) **EP 2 237 030 A1**
(43) Date of publication of application: **06.10.2010**
(21) Application number: 09700361.0
(22) Date of filing: 09.01.2009
(51) Int. Cl.: G01N 27/416, G01N 33/543, C12N 15/09

(54) **METHOD FOR SPECIFICALLY DETECTING TEST SUBSTANCE USING PHOTOCURRENT, SENSOR UNIT USED THEREFOR, AND MEASURING DEVICE**

(30) Priority: 09.01.2008 JP 2008002530
(71) Applicant: Toto Ltd., Fukuoka 802-8601 (JP)
(72) Inventor: BEKKI, Makoto, Kitakyushu-shi Fukuoka 802-8601 (JP); AJIMI, Masako, Kitakyushu-shi Fukuoka 802-8601 (JP); OHARA, Hitoshi, Kitakyushu-shi Fukuoka 802-8601 (JP); SONEZAKI, Shuji, Kitakyushu-shi Fukuoka 802-8601 (JP); YAMANA, Yoshimasa, Kitakyushu-shi Fukuoka 802-8601 (JP)
(74) Representative: ABG Patentes, S.L.
(86) International application number: PCT/JP2009/050245
(87) International publication number: WO 2009/088082

(57) **Abstract**

This invention provides a method for detecting an analyte, which, in specific detection of an analyte using photocurrent that is generated by photoexcitation of a sensitizing dye, can significantly simplify the structure and a detection procedure of a sensor unit and a device using the sensor unit and can realize detection with high accuracy, and also provides a sensor unit and a device using the sensor unit. According to the method, an analyte-containing sample solution and a sensitizing dye are brought into contact with the surface of a working electrode to immobilize the sensitizing dye onto the working electrode through specific binding. Subsequently, an electrolyte is supplied in situ while allowing the sample solution to be held without being removed to bring the working electrode and a counter electrode into contact with the electrolyte. The working electrode is irradiated with light to potoexcite the sensitizing dye and thus to detect photocurrent that flows across the working electrode and the counter electrode.

SEQUENCE LISTING

<110>  TOTO Ltd.

<120>  Method for Specifically Detecting Test Substance Using Photocurrent,
Sensor Unit and Measuring Apparatus Used Therefor

<130>  177358PX

<150> JP 2008-002530
<151> 2008-01-09

<160>  2

<170>  PatentIn version 3.1

<210>  1
<211>  25
<212>  DNA
<213>  Artificial

<220>
<223>  probe

<400>  1
gcggcatgaa cctgaggccc atcct                                          25

<210>  2
<211>  24
<212>  DNA
<213>  Artificial

<220>
<223>  probe

<400> 2

ttgagcaagt tcagcctggt taag 24

## Description

### BACKGROUND OF THE INVENTION

### Field of Invention

The present invention relates to a method for specifically detecting analytes having a specific binding capability such as nucleic acids, endocrine disrupting chemicals, or antigens through the utilization of photocurrent, and a sensor unit and a measuring device using the method.

### Background Art

Present-day attempts have been made to develop testing systems that can simply detect at low cost hormones and proteins which can predict the symptom and progress of diseases of humans. Further, damage to genital systems, nervous systems and the like by endocrine disrupting chemicals (environmental hormones) including dioxins is recognized as social problems, leading to a demand for the development of a simple method that can detect endocrine disrupting chemicals.

For example, a proposal has been made on the utilization of photocurrent generated by photoexcitation of a sensitizing dye in the detection of analytes (biomolecules such as DNAs and proteins) utilizing the principle of solar cells that generate electric energy from light using a sensitizing dye (see, for example, Japanese Patent 2002-181777 A, Japanese Patent 2005-251426 A, Japanese Patent 2006-119111 A, and Japanese Patent 2006-507491 T). According to this method, analytes can be relatively simply detected using photocurrent. However, a further improvement in simplicity and accuracy has been desired.

Further, methods have been carried out in which the principle of immunochromatography is used to electrochemically detect analytes such as hormones, proteins, and enzymes (see, for example, Japanese Patent 2001-337065 A, Japanese Patent H08-327582 A, and Japanese Patent 2006-524815 T). According to these methods, an analyte can be relatively easily detected using a simple device construction. Since, however, a labeled analyte remaining unbound should be separated and removed utilizing the flow of a sample solution through a capillary phenomenon of an absorption pad, the time taken from spot sticking to detection of the analyte is disadvantageously long.

### SUMMARY OF THE INVENTION

The present inventors have now found that, in specific detection of an analyte using photocurrent generated by photoexcitation of a sensitizing dye, the supply of an electrolyte in situ while holding a provided analyte-containing sample solution without removing the sample solution can realize the detection of the analyte with high accuracy while significantly simplifying the structure of a sensor unit and a device using the sensor unit and a detection procedure.

Accordingly, an object of the present invention is to provide a method for detecting an analyte, which, in specific detection of an analyte using photocurrent generated by photoexcitation of a sensitizing dye, can detect the analyte with high accuracy while significantly simplifying the structure of a sensor unit and a device using the sensor unit and a detection procedure, and to provide a sensor unit and a device using the sensor unit.

According to the present invention, there is provided a method for specifically detecting an analyte using photocurrent that flows, across a working electrode and a counter electrode, attributable to transfer of electrons from a photoexcited sensitizing dye to a working electrode, the working electrode having on its surface a probe substance capable of specifically binding directly or indirectly to the analyte, the method comprising:
bringing an analyte-containing sample solution and the sensitizing dye into contact with the surface of the working electrode to immobilize the sensitizing dye to the working electrode through specific binding;
supplying an electrolyte in situ while allowing the sample solution to be held without being removed, thereby bringing the working electrode and the counter electrode into contact with the electrolyte; and
irradiating the working electrode with light to photoexcite the sensitizing dye and thus to detect photocurrent that flows across the working electrode and the counter electrode.

According to another aspect of the present invention, there is provided a sensor unit for use in a method for specifically detecting an analyte using photocurrent that flows, across a working electrode and a counter electrode, attributable to transfer of electrons from a photoexcited sensitizing dye to a working electrode, the sensor unit comprising:
a working electrode that has on its surface a probe substance capable of specifically binding directly or indirectly to the analyte and can allow the sensitizing dye to be immobilized thereonto through specific binding by the contact of the working electrode with an analyte-containing sample solution and the sensitizing dye;
a counter electrode; and
a sample solution holding member that does not have a mechanism for removing the sample solution and holds the sample solution on the surface of the working electrode and the counter electrode.

According to a further aspect of the present invention, there is provided a measuring device for use in a method for specifically detecting an analyte using photocurrent that flows, across a working electrode and a counter electrode, attributable to transfer of electrons from a photoexcited sensitizing dye to a working electrode, the measuring device comprising:
the above sensor unit;
a light source that applies light to the working electrode; and
an ammeter that detects photocurrent that flows, across the working electrode and the counter electrode, attributable to the transfer of electrons from the photoexcited sensitizing dye to the working electrode.

### BRIEF DESCRIPTION OF DRAWINGS

[Fig. 1] Fig. 1 is a typical exploded view of a sensor unit in a first embodiment of the present invention.
[Fig. 2] Fig. 2 is a cross-sectional view of the sensor unit shown in Fig. 1 in a specific reaction.
[Fig. 3] Fig. 3 is a cross-sectional view of the sensor unit shown in Fig. 1 in the detection of photocurrent.
[Fig. 4] Fig. 4 is a typical exploded view of a variant of the sensor unit in the first embodiment of the present invention.
[Fig. 5] Fig. 5 is a cross-sectional view of the sensor unit shown in Fig. 4 in a specific reaction.
[Fig. 6] Fig. 6 is a cross-sectional view of the sensor unit shown in Fig. 4 in the detection of photocurrent.
[Fig. 7] Fig. 7 is a typical exploded view of a sensor unit in a second embodiment of the present invention.
[Fig. 8] Fig. 8 is a cross-sectional view of the sensor unit shown in Fig. 7 in a specific reaction.
[Fig. 9] Fig. 9 is a cross-sectional view of the sensor unit shown in Fig. 7 in the detection of photocurrent.
[Fig. 10] Fig. 10 is a typical exploded view of a variant of the sensor unit in the second embodiment of the present invention.
[Fig. 11] Fig. 11 is a cross-sectional view of the sensor unit shown in Fig. 10 in a specific reaction.
[Fig. 12] Fig. 12 is a cross-sectional view of the sensor unit shown in Fig. 10 in the detection of photocurrent.
[Fig. 13] Fig. 13 is a typical exploded view of a sensor unit in a third embodiment of the present invention.
[Fig. 14] Fig. 14 is a cross-sectional view of the sensor unit shown in Fig. 13 in a specific reaction.
[Fig. 15] Fig. 15 is a cross-sectional view of the sensor unit shown in Fig. 13 in the detection of photocurrent.
[Fig. 16] Fig. 16 is a typical exploded view of a variant of the sensor unit in the third embodiment of the present invention.
[Fig. 17] Fig. 17 is a cross-sectional view of the sensor unit shown in Fig. 16 in a specific reaction.
[Fig. 18] Fig. 18 is a cross-sectional view of the sensor unit shown in Fig. 16 in the detection of photocurrent.
[Fig. 19] Fig. 19 is a typical exploded view of a sensor unit in a fourth embodiment of the present invention.
[Fig. 20] Fig. 20 is a cross-sectional view of the sensor unit shown in Fig. 19 in a specific reaction.
[Fig. 21] Fig. 21 is a cross-sectional view of the sensor unit shown in Fig. 19 in the detection of photocurrent.
[Fig. 22] Fig. 22 is a typical exploded view of a variant of the sensor unit in the fourth embodiment of the present invention.
[Fig. 23] Fig. 23 is a cross-sectional view of the sensor unit shown in Fig. 22 in a specific reaction.
[Fig. 24] Fig. 24 is a cross-sectional view of the sensor unit shown in Fig. 22 in the detection of photocurrent.
[Fig. 25] Fig. 25 is a typical view illustrating the step of immobilizing an antigen as an analyte and a sensitizing dye-labeled secondary antibody onto a primary antibody as a probe substance in immunoassay, particularly a sandwich method.
[Fig. 26] Fig. 26 is a typical view illustrating the step of immobilizing an antigen as an analyte and an antigen as a sensitizing dye-labeled second analyte onto an antibody as a probe substance in competitive immunoassay.
[Fig. 27] Fig. 27 is a typical view illustrating the step of immobilizing a ligand as an analyte onto a working electrode in receptor binding assay.
[Fig. 28] Fig. 28 is a typical view illustrating the step of immobilizing an antigen as an analyte onto a working electrode in immunoassay by an open sandwich method.
[Fig. 29] Fig. 29 is a typical view of an electrode unit in one embodiment of the present invention.
[Fig. 30] Fig. 30 is a typical exploded view of a sensor unit in one embodiment of the present invention, which has been used in the detection of photocurrent using an electrolyte pad in Example 1.
[Fig. 31] Fig. 31 is a typical exploded view of a flow cell-type measuring cell, which has been used in the detection of photocurrent using an electrolyte solution in Example 1.
[Fig. 32] Fig. 32 is a graph showing photocurrent measured using an immersion electrolyte pad, a condensate electrolyte pad, and an electrolyte solution in Example 1.
[Fig. 33] Fig. 33 is a graph showing photocurrent measured using a reaction pad and a spacer in Example 2.
[Fig. 34] Fig. 34 is a graph showing photocurrent measured using a heat-dried reaction pad and a lyophilized reaction pad in Example 3.
[Fig. 35] Fig. 35 is a graph showing photocurrent measured for the case where an electrolyte is present in a reaction solution and for the case where an electrolyte is absent in a reaction solution in Example 4.
[Fig. 36] Fig. 36 is a graph showing photocurrent measured using different reaction pads in Example 5.
[Fig. 37] Fig. 37 is a graph showing photocurrent measured using different electrolyte solutions in Example 6.
[Fig. 38] Fig. 38 is a graph showing photocurrent measured for different sample solution holding members and different electrolyte supply forms in Example 7.
[Fig. 39] Fig. 39 is a graph showing the relationship between the concentration of tetrapropylammonium iodide and the photocurrent, measured in Example 8.
[Fig. 40] Fig. 40 is a graph showing an increase in photocurrent dependent upon the amount of immobilized ssDNA, measured for different electrolytes in Example 8.
[Fig. 41] Fig. 41 is a graph showing the relationship between the thickness of an electrolyte-containing pad and photocurrent, measured in Example 8.
[Fig. 42] Fig. 42 is a graph showing an increase in photocurrent dependent upon the amount of immobilized ssDNA, measured for different water absorptive substances in Example 8.
[Fig. 43] Fig. 43 is a graph showing the relationship between the water content of an electrolyte-containing pad and photocurrent, measured in Example 8.
[Fig. 44] Fig. 44 is a typical exploded view of a sensor unit in one embodiment of the present invention used in Example 9.
[Fig. 45] Fig. 45 is a cross-sectional view of the sensor unit shown in Fig. 44 in the detection of photocurrent.
[Fig. 46] Fig. 46 is a graph showing photocurrent measured in Example 9.
[Fig. 47] Fig. 47 is a typical exploded view of a sensor unit in one embodiment of the present invention used in Example 10.
[Fig. 48] Fig. 48 is a cross-sectional view of the sensor unit shown in Fig. 47 in the detection of photocurrent.
[Fig. 49] Fig. 49 is a graph showing photocurrent measured in Example 10.

### DETAILED DESCRIPTION OF THE INVENTION

### Detection method, sensor unit, and measuring device

The method for specifically detecting an analyte according to the present invention is carried out using photocurrent that flow, across a working electrode and a counter electrode, attributable to the transfer of electrons from a photoexcited sensitizing dye to the working electrode. The working electrode has on its surface a probe substance capable of specifically binding directly or indirectly to the analyte. In the method according to the present invention, an analyte-containing sample solution and a sensitizing dye are brought into contact with the surface of the working electrode to immobilize the sensitizing dye to the working electrode through direct or indirect specific binding between the analyte and the probe substance. Subsequently, an electrolyte is supplied in situ while allowing the sample solution to be held without being removed, thereby bringing the working electrode and the counter electrode into contact with the electrolyte. The working electrode is irradiated with light to photoexcite the sensitizing dye and thus to detect photocurrent that flows across the working electrode and the counter electrode. The sensor unit according to the present invention which is suitable for use in the detection method according to the present invention comprises a working electrode, a counter electrode, and a sample solution holding member that does not have a mechanism for removing the sample solution and holds the sample solution on the surface of the working electrode and the counter electrode. The sensor unit according to the present invention is not limited to use in the method according to the present invention and can be widely used in the method for specifically detecting an analyte using photocurrent that flows, across a working electrode and a counter electrode, attributable to the transfer of electrons from a photoexcited sensitizing dye to a working electrode.

Thus, the method and sensor unit according to the present invention are characterized in that, after the immobilization of an analyte and a sensitizing dye onto a working electrode, an electrolyte can be supplied in situ while holding a sample solution without removing the sample solution to perform measurement. In the method for specifically detecting an analyte by the conventional method, after the immobilization of an analyte and a sensitizing dye onto a working electrode and before subsequent photocurrent measurement, a sample solution containing the residual unbound analyte and sensitizing dye has been once removed by washing and streams. It is understood based on common recognition that substances, present as a mixture, such as the residual unbound analyte or sensitizing dye, salts, or surfactants possibly lower the detection accuracy of the analyte. The present inventors, however, have unexpectedly found that, even when the sample solution containing substances, present as a mixture, such as the residual unbound analyte or sensitizing dye, salts or surfactants is not removed, photocurrent derived from these residual substances is hardly detected and the detection can be carried out with high accuracy. As a result, in the specific detection of the analyte using photocurrent generated by photoexcitation of the sensitizing dye, the structure of the sensor unit and the device using the sensor unit and a detection procedure could have been significantly simplified and detection with high accuracy has become possible.

In particular, as described above, specific detection of the analyte using photocurrent has hitherto been carried out using a sensor unit filled with an electrolyte solution. This method poses problems involved in a solution feeding mechanism that feeds a solution into the sensor unit, for example, time loss caused by solution feeding, an increase in complexity and an increase in size of the device, and solution leakage. According to the present invention not involving the removal of the sample solution, these problems are reduced. Further, in the present invention, since the removal of the sample solution is unnecessary, unlike the conventional immunochromatography that requires a lot of time in the separation and removal of the labeled analyte, the absorption pad for chromatography used is unnecessary, contributing to significant simplification of the structure of the sensor unit and the device using the sensor unit and a detection procedure. Further, the method according to the present invention is advantageous in that the addition of an electrolyte after the immobilization of the analyte and the sensitizing dye onto the working electrode can realize a specific reaction between the analyte and the probe substance with high efficiency and can improve the accuracy of detection of the analyte.

In the method according to the present invention, at the outset, an analyte-containing sample solution and the sensitizing dye are brought into contact with the surface of the working electrode to immobilize the sensitizing dye to the working electrode through specific binding. The working electrode has on its surface a probe substance capable of specifically binding directly or indirectly to the analyte. The sample solution, the sensitizing dye, the working electrode, the counter electrode, the probe substance and the like usable in the present invention will be described later.

While holding the sample solution without removing the sample solution, an electrolyte is supplied in situ to bring the working electrode and the counter electrode into contact with the electrolyte. Thus, even when the sample solution containing substances, present as a mixture, such as the residual unbound analyte or sensitizing dye, salts or surfactants is not removed, photocurrent derived from these residual substances is hardly detected and the detection can be carried out with high accuracy. As a result, in the specific detection of the analyte using photocurrent generated by photoexcitation of the sensitizing dye, the structure of the sensor unit and the device using the sensor unit and a detection procedure can be significantly simplified and the detection can be carried out with high accuracy. Accordingly, the sample solution holding member used in the present invention may be any member that can hold the sample solution on the surface of the working electrode and the counter electrode, as long as the member does not have a mechanism for removing the sample solution.

In a preferred embodiment of the present invention, the contact of the surface of the working electrode with the sample solution can be carried out through a spacer having an opening for holding the sample solution on the surface of the working electrode. That is, the sample solution holding member may be a spacer having an opening fillable with the sample solution. In the present invention, the spacer is used to add a sample solution containing the analyte and the sensitizing dye onto the working electrode and thus is preferably formed of an insulating material such as rubber or other synthetic resins so that the working electrode and the counter electrode are not electrically connected to each other. Examples of insulating materials usable herein include nitrile rubber, fluoro rubber, urethane rubber, silicone rubber, ethylene propylene rubber, hydrogenated nitrile rubber, chloroprene rubber, acrylic rubber, butyl rubber, polyvinyl chloride, polyethylene terephthalate, polyester, polycarbonate, polystyrene, and cellophane. A sheet formed of the above insulating material is suitable as the spacer used in the present invention. Specific binding between the analyte and the probe substance can be carried out within the opening in the spacer by adding a sample solution containing the analyte into the opening in the spacer.

In another preferred embodiment of the present invention, the contact of the surface of the working electrode with the sample solution can be carried out through a water-absorptive reaction pad for holding the reaction solution on the surface of the working electrode. That is, the sample solution holding member may be a water-absorptive reaction pad that can be impregnated with the sample solution. The reaction pad used in the present invention is used as a medium for the analyte and the sensitizing dye in the specific detection of the analyte using photocurrent generated by the photoexcitation of the sensitizing dye. The impregnation of the reaction pad with the analyte-containing sample solution can allow the analyte to be specifically bound to the probe substance on the reaction pad. The adoption of the reaction pad makes it easy to handle the sample solution without posing a problem such as solution leakage and thus can effectively simplify the structure of the sensor unit and the device using the sensor unit and a detection procedure.

In a preferred embodiment of the present invention, the electrolyte can be supplied by adding an electrolyte solution. Preferably, the electrolyte solution comprises an electrolyte of a salt which can supply electrons to an oxidized sensitizing dye, and at least one solvent selected from aprotic solvents and protic solvents.

In another preferred embodiment of the present invention, the electrolyte may be supplied by previously impregnating the water-absorptive electrolyte pad with the electrolyte and bringing the electrolyte-impregnated water-absorptive electrolyte pad into contact with the sample solution to diffuse the electrolyte into the sample solution. That is, in the present invention, in the specific detection of an analyte using photocurrent generated by photoexcitation of the sensitizing dye, an electrolyte pad may be used instead of the electrolyte solution. Further, the electrolyte pad according to the present invention is used as a medium for the electrolyte. Preferably, the electrolyte pad is provided at a position that is away from the sample solution holding member in its area, where the sample solution is to be held, and is on the opposite side of the working electrode and so that, after specific binding, the electrolyte pad can be brought into contact with the area where the sample solution is held. The adoption of the electrolyte pad makes it easy to supply the electrolyte without posing a problem such as solution leakage and thus can effectively simplify the structure of the sensor unit and the device using the sensor unit and a detection procedure.

A wide variety of electrolytes may be used as the electrolyte in the present invention without limitation, as long as the electrolyte can be freely moved in the reaction pad or the electrolyte pad to participate in supply and receipt of electrons among the sensitizing dye, the working electrode, and the counter electrode. Preferred electrolytes are substances that can function as a reducing agent (an electron donating agent) for supplying electrons to a dye photoexcited by light irradiation. Examples of such substances include sodium iodide (NaI), potassium iodide (KI), calcium iodide (CaI₂), lithium iodide (LiI), ammonium iodide (NH₄I), tetrapropylammonium iodide (NPr₄I), sodium thiosulfate (Na₂S₂O₃), sodium sulfite (Na₂SO₃), hydroquinone, K₄[Fe(CN)₆]·3H₂O, ferrocene-1,1'-dicarboxylic acid, ferrocene carboxylic acid, sodium borohydride (NaBH₄), triethylamine, thiocyanate ammonium, hydrazine (N₂H₄), acetaldehyde (CH₃CHO), N,N,N',N'-tetramethyl-p-phenylenediamine dichloride (TMPD), L-ascorbic acid, sodium tellurite (Na₂TeO₃), iron(II) chloride tetrahydrate (FeCl₂·4H₂O), EDTA, cysteine, triethanolamine, tripropylamine, iodine-containing lithium iodide (I/LiI), tris(2-chloroethyl)phosphate (TCEP), dithiothreitol (DTT), 2-mercaptoethanol, 2-mercaptoethanolamine, thiourea dioxide, (COOH)₂, HCHO, and combinations thereof. More preferred are Nal, KI, CaI₂, LiI, NH₄I, tetrapropylammonium iodide (NPr₄I), sodium thiosulfate (Na₂S₂O₃), sodium sulfite (Na₂SO₃), and mixtures thereof. Still more preferred are tetrapropylammonium iodide (NPr₄I).

In a preferred embodiment of the present invention, in detecting photocurrent, the reaction pad and/or the electrolyte pad have a water content of not less than 20%, more preferably not less than 30%, still more preferably not less than 40%. When the water content is in the above-defined range, high photocurrent can be detected in the detection of photocurrent, contributing to improved detection sensitivity. The water content is determined from (amount of water per mm³)/(density of water-absorptive substance). The water content referred to herein is the water content of the pad in detecting photocurrent and may not satisfy the above water content before the detection of photocurrent.

Preferably, the reaction pad and/or the electrolyte pad according to the present invention have a smooth plane area of contact with each electrode from the viewpoint of ensuring good adhesion to the working electrode and the counter electrode. Accordingly, in use, when these pads are held between the working electrode and the counter electrode, preferably, they have a uniform thickness so as not to affect the adhesion. On the other hand, when an electrode unit comprising a working electrode and a counter electron patterned on the same plane is used, the adoption of a smooth plane at least only in one side surface which comes into contact with the electrode unit suffices for contemplated results and, in this case, the thickness and the uniformity of the thickness pose no particular problem.

In a preferred embodiment of the present invention, the reaction pad and/or the electrolyte pad have a thickness of 0.01 to 10 mm, more preferably 0.1 to 3 mm. The thickness in the above-defined range can easily provide strength suitable for sole handling of the pads, and, hence, the pads can easily be held between the working electrode and the counter electrode, can easily be removed, or can also be carried. As a result, the structure of the sensor unit and a detection procedure can be significantly simplified. Further, adverse effect on the measurement of photocurrent can be avoided.

In a preferred embodiment of the present invention, the reaction pad and/or the electrolyte pad contain a water-absorptive substance. Examples of such water-absorptive substances include natural fibers such as cotton, hemp, wool, silk, and cellulose; pulp fibers used for filter paper, papermaking and the like; recycled fibers such as rayon; glass fibers used for filter paper and the like; synthetic fibers used for felts, sponges and the like; and combinations thereof. The water-absorptive substance is not limited as long as the water-absorptive substance exhibits appropriate strength, appropriate water content, and appropriate adhesion to the electrode. The fiber processing method is not limited to a particular processing method.

In a preferred embodiment of the present invention, the reaction pad and/or the electrolyte pad are in a sheet form. Preferred examples thereof include filter papers, membrane filters, glass filters, and filter clothes. More preferred are filter papers and membrane filters.

In a preferred embodiment of the present invention, a barrier film for blocking the diffusion of the electrolyte from the electrolyte pad into the reaction pad is previously provided between the reaction pad and the electrolyte pad and the barrier film is removed when the electrolyte is supplied. In the present invention, the barrier film is used in order to prevent the sample solution from being interfused into the electrolyte pad when the sample solution containing the analyte and the sensitizing dye is added onto the reaction pad in contact with the working electrode. The barrier film may be formed of any material as long as the solution is interfused thereinto. Examples of such materials include polyesters and polypropylene. In particular, in the method according to the present invention, when an electrolyte is added after the immobilization of the analyte and the sensitizing dye onto the working electrode, the analyte can be specifically reacted with the probe substance with high efficiency to improve the detection accuracy of the analyte. According to the construction using the barrier film, the post addition of the electrolyte can be efficiently realized.

The lamination of the working electrode, the counter electrode, the sample solution holding member, and, if any, the electrolyte pad as described above can construct a sensor unit. In the sensor unit, the working electrode is irradiated with light to photoexcite the sensitizing dye and to detect photocurrent that flows across the working electrode and the counter electrode. At least when light is applied, the working electrode and the counter electrode may be disposed so as to face each other, or alternatively the working electrode and the counter electrode may be disposed on the same plane of an insulating substrate or the like. The relative positional relationship between the working electrode and the counter electrode is not particularly limited. When the working electrode and the counter electrode are disposed so as to face each other, preferably, a sample solution holding member and, if any, an electrolyte pad are held between the working electrode and the counter electrode. On the other hand, when the working electrode and the counter electrode are disposed on the same plane, a sample solution holding member is preferably disposed in contact with the plane. In a preferred embodiment of the present invention, a pressing member and a counter member are provided so as to face each other in order to hold the laminate of the working electrode, the counter electrode, the sample solution holding member, and, if desired, the electrolyte pad therebetween.

The measuring device according to the present invention is constructed by further providing, on the sensor unit, a light source for applying light to the working electrode and an ammeter for detecting photocurrent that flows, across the working electrode and the counter electrode, attributable to the transfer of electrons from the photoexcited sensitizing dye to the working electrode.

### Sensor unit and measuring device in preferred embodiment

As described above, the use of the sensor unit according to the present invention can realize the construction of a sensor unit or a measuring device that has a significantly simplified structure, is low in cost, and has a small size. This is because the use of the sensor unit according to the present invention can eliminate the need to provide complicated mechanisms or processes, such as a mechanism for supplying an electrolyte solution (for example, a pump, a valve, and a mechanism for controlling them), a mechanism for preventing liquid leakage (for example, packing) and waste liquid treatment of the electrolyte solution, which were required in conventional methods that are carried out by filling an electrolyte solution into between the working electrode and the counter electrode, and, further, a very simple reactor mechanism can be realized by performing specific binding of the analyte on the reaction pad or within the spacer. Preferred embodiments of the sensor unit according to the present invention will be described.

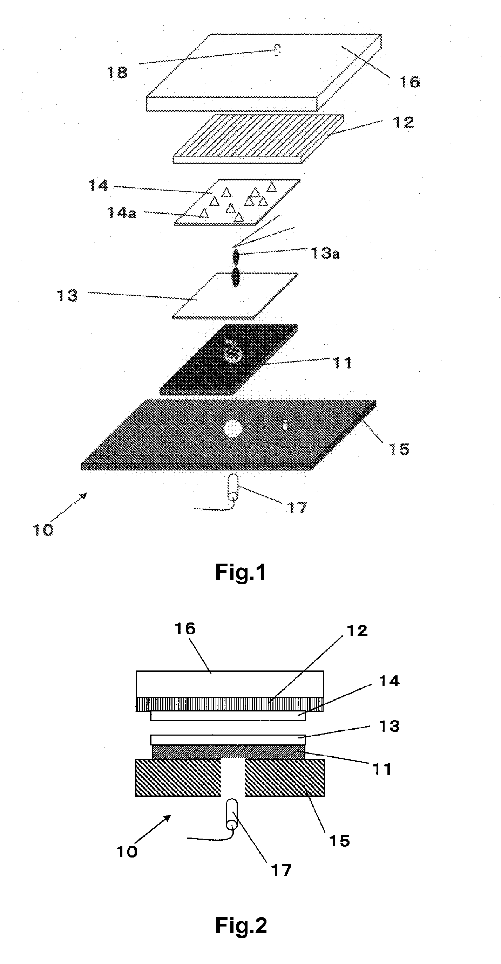

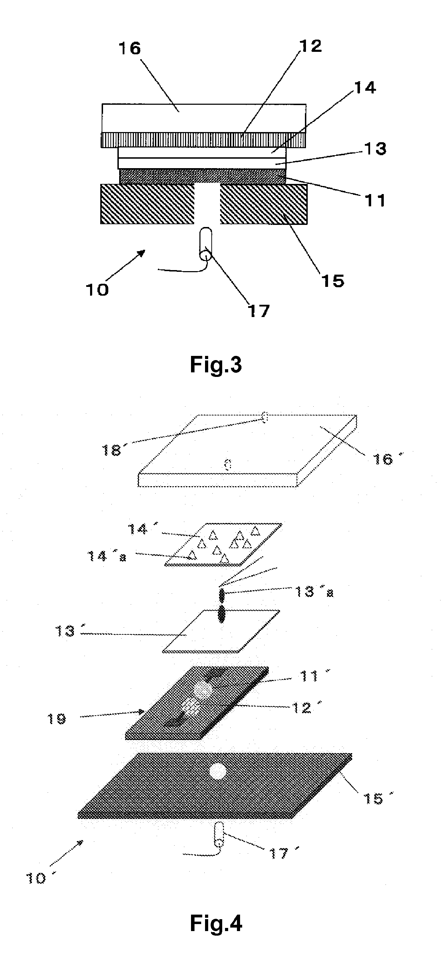

The sensor unit in the first embodiment of the present invention comprises a working electrode, a counter electrode, a reaction pad held between the working electrode and the counter electrode, and an electrolyte pad. In the specific reaction, the working electrode comes into contact with the sample solution-added reaction pad, and, in the detection of photocurrent, the electrolyte pad is brought into contact with the reaction pad to allow the electrolyte diffused in the reaction pad to participate in the supply and receipt of electrons among the sensitizing dye, the working electrode, and the counter electrode. That is, in the specific reaction, the working electrode is brought into contact with the sample solution-added reaction pad, and, after the completion of the step of the specific reaction, the electrolyte pad is brought into contact with the reaction pad to detect current. Figs. 1, 2, and 3 are respectively a typical exploded view of a sensor unit in the first embodiment, a cross-sectional view of the sensor unit in the specific reaction, and a cross-sectional view of the sensor unit in the detection of current. In Fig. 1, a sensor unit 10 basically comprises a working electrode 11, a counter electrode 12, a reaction pad 13, and an electrolyte pad 14 and further comprises a pressing member 15 that supports the working electrode 11. A counter member 16 is provided on the uppermost part of the sensor unit 10 to press downward the working electrode 11, the reaction pad 13, the electrolyte pad 14, and the counter electrode 12 stacked in that order on the pressing member 15 to bring them into close contact with each other. In the specific reaction, as shown in Fig. 2, the working electrode 11 and the reaction pad 13 to which a sample solution 13a has been added are brought into contact with each other. Although the reaction pad 13 and the electrolyte pad 14 are provided away from each other, in the detection of current, as shown in Fig. 3, the reaction pad 13 and the electrolyte pad 14 are brought into contact with each other to allow an electrolyte 14a contained in the electrolyte pad 14 to participate in the supply and receipt of electrons among the sensitizing dye, the working electrode, and the counter electrode.

In the sensor unit of the present invention, the order of components to be stacked is not limited to the embodiment shown in the drawing, and the components may be stacked in the reverse order of the order shown in Fig. 1 to turn the laminate upside down. In this case, a construction is adopted in which a counter member for supporting the counter electrode and a pressing member for pressing downward the counter electrode, the electrolyte pad, the reaction pad, and the working electrode stacked in that order on the counter member to bring them into close contact with each other are provided. In the embodiment shown in the drawing, the members are horizontally arranged, but may be arranged in an upright state. Preferably, the pressing member 15 further comprises an opening or a light-transparent part that allows light for photoexcitation from the light source 17 to be passed thereinto.

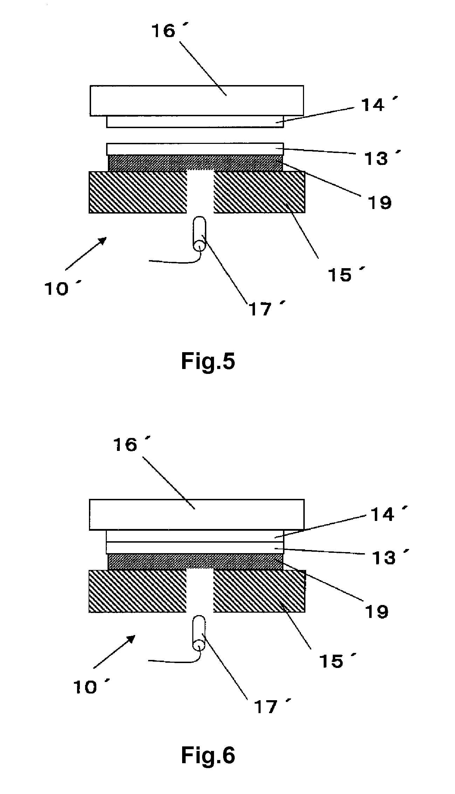

Figs. 4, 5, and 6 are respectively a typical exploded view of a sensor unit 10' using an electrode unit comprising a working electrode 11' and a counter electrode 12' patterned on the same plane in the sensor unit shown in Figs. 1 to 3, a cross-sectional view of the sensor unit 10' in the specific reaction, and a cross-sectional view of the sensor unit 10' in the detection of current, which are a variant of the sensor unit in the first embodiment. In the sensor unit 10' shown in Figs. 4 to 6, a counter member 16' is provided so as to face an electrode unit 19, and a reaction pad 13' and an electrolyte pad 14' are held between the electrode unit 19 and the counter member 16'. The sensor unit 10' further comprises a pressing member 15' that supports the electrode unit 19. That is, the electrode unit 19 is disposed on the pressing member 15' so that the analyte-immobilized side faces upward. In the specific reaction, as shown in Fig. 5, the electrode unit 19 is brought into contact with the reaction pad 13' to which the sample solution 13'a has been added. Although the reaction pad 13' and the electrolyte pad 14' are away from each other, in the detection of current, as shown in Fig. 6, the reaction pad 13' and the electrolyte pad 14' are brought into contact with each other to allow the electrolyte 14'a contained in the electrolyte pad 14' to participate in the supply and receipt of electrons among the sensitizing dye, the working electrode, and the counter electrode. A contact for connecting the working electrode to the ammeter is preferably ensured by a contact probe 18 in the sensor unit shown in Figs. 1 to 3 and by a contact probe 18' in the sensor unit shown in Figs. 4 to 6. This is true of the counter electrode.

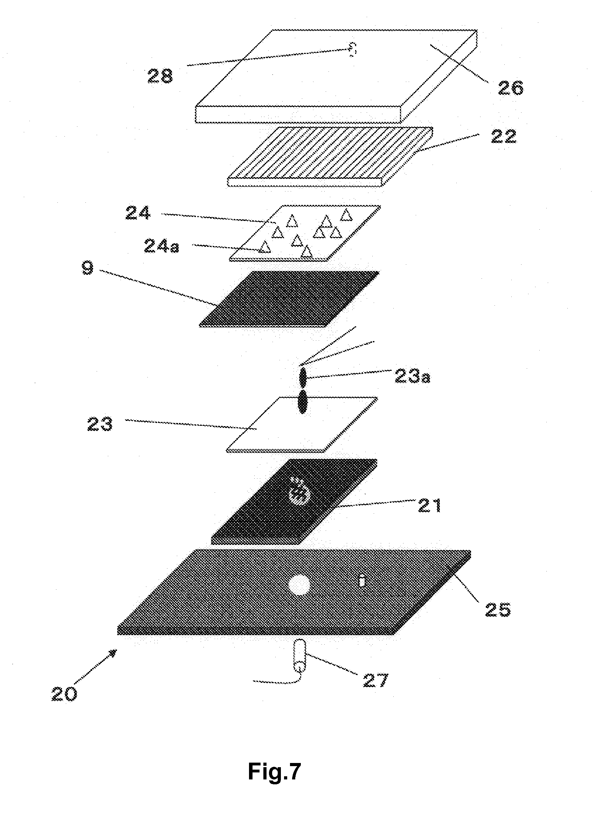

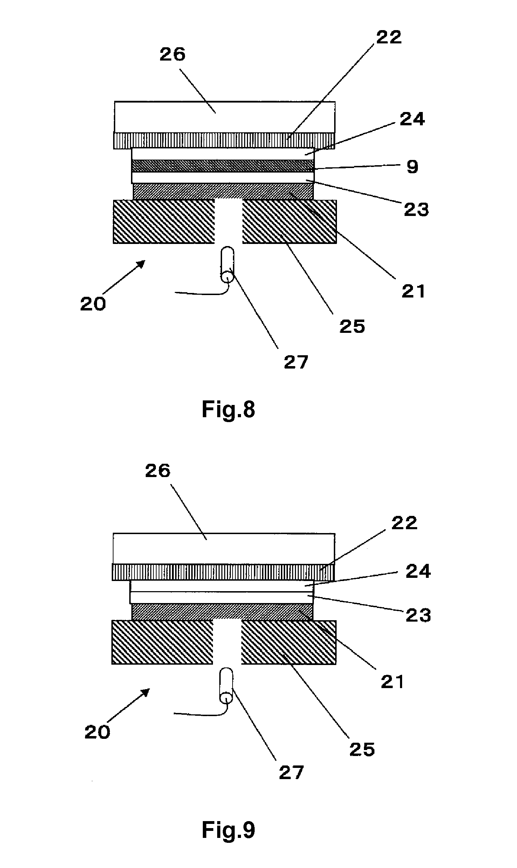

The sensor unit in the second embodiment of the present invention comprises a working electrode, a counter electrode, a reaction pad held between the working electrode and the counter electrode, a barrier film, and an electrolyte pad. In the specific reaction, the working electrode is brought into contact with the sample solution-added reaction pad, and, in the detection of current, the barrier film is removed to bring the electrolyte pad into contact with the reaction pad to allow the electrolyte diffused in the reaction pad to participate in the supply and receipt of electrons among the sensitizing dye, the working electrode, and the counter electrode. That is, in the specific reaction, the working electrode is brought into contact with the sample solution-added reaction pad, and, after the completion of the step of the specific reaction, the barrier film is removed to bring the electrolyte pad into contact with the reaction pad to detect current. Figs. 7, 8, and 9 are respectively a typical exploded view of a sensor unit in the second embodiment, a cross-sectional view of the sensor unit in the specific reaction, and a cross-sectional view of the sensor unit in the detection of current. In Fig. 7, a sensor unit 20 basically comprises a working electrode 21, a counter electrode 22, a reaction pad 23, a barrier film 9, and an electrolyte pad 24 and further comprises a pressing member 25 that supports the working electrode 21. A counter member 26 is provided on the uppermost part of the sensor unit 20 to press downward the working electrode 21, the reaction pad 23, the barrier film 9, the electrolyte pad 24, and the counter electrode 22 stacked in that order on the pressing member 25 to bring them into close contact with each other. In the specific reaction, as shown in Fig. 8, the working electrode 21 and the reaction pad 23 to which a sample solution 23a has been added are brought into contact with each other. Although the reaction pad 23 and the electrolyte pad 24 are provided away from each other through the barrier film 9, in the detection of current, as shown in Fig. 9, the reaction pad 23 and the electrolyte pad 24 are brought into contact with each other by removing the barrier film 9 to allow an electrolyte 24a contained in the electrolyte pad 24 to participate in the supply and receipt of electrons among the sensitizing dye, the working electrode, and the counter electrode.

In the sensor unit of the present invention, the order of components to be stacked is not limited to the embodiment shown in the drawing, and the components may be stacked in the reverse order of the order shown in Fig. 3 to turn the laminate upside down. In this case, a construction is adopted in which a counter member for supporting the counter electrode and a pressing member for pressing the counter electrode, the electrolyte pad, the barrier film, the reaction pad, and the working electrode stacked in that order on the counter member to bring them into close contact with each other are provided. In the embodiment shown in the drawing, the members are horizontally arranged, but may be arranged in an upright state. Preferably, the pressing member 25 further comprises an opening or a light-transparent part that allows light for photoexcitation from the light source 27 to be passed thereinto.

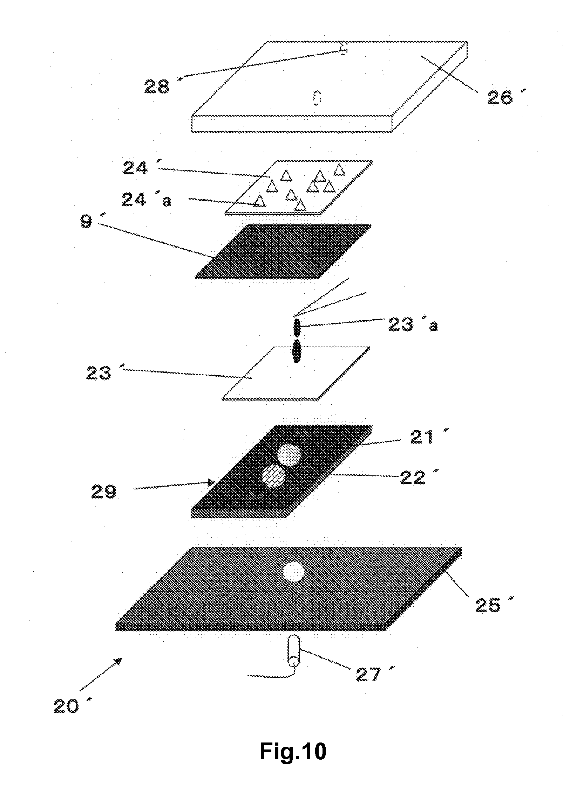

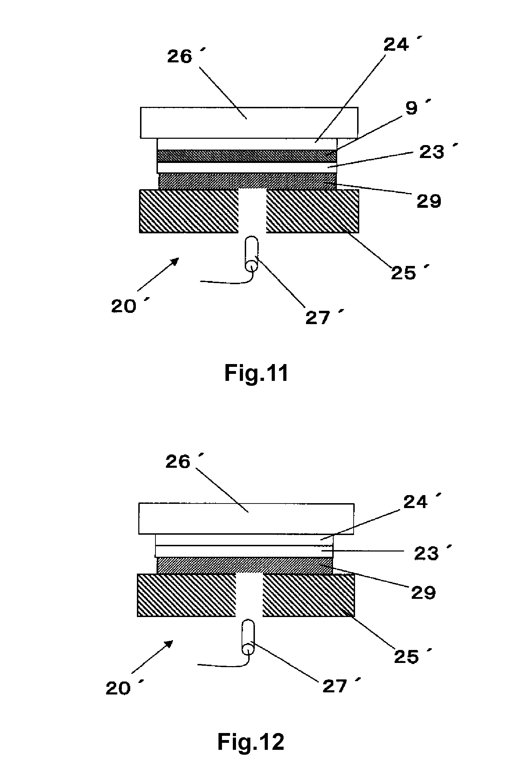

Figs. 10, 11, and 12 are respectively a typical exploded view of a sensor unit 10' using an electrode unit comprising a working electrode 21' and a counter electrode 22' patterned on the same plane in the sensor unit shown in Figs. 7 to 9, a cross-sectional view of the sensor unit 10' in the specific reaction, and a cross-sectional view of the sensor unit 10' in the detection of current, which are a variant of the sensor unit in the second embodiment. In the sensor unit 20' shown in Fig. 10, a counter member 26' is provided so as to face an electrode unit 29, and a reaction pad 23', a barrier film 9', and an electrolyte pad 24' are held between the electrode unit 29 and the counter member 26'. The sensor unit 20' further comprises a pressing member 25' that supports the electrode unit 29. That is, the electrode unit 29 is disposed on the pressing member 25' so that the analyte-immobilized side faces upward. In the specific reaction, as shown in Fig. 11, the electrode unit 29 is brought into contact with the reaction pad 23' to which the sample solution 23'a has been added. Although the reaction pad 23' and the electrolyte pad 24' are away from each other through the barrier film 9', in the detection of current, as shown in Fig. 12, the reaction pad 23' and the electrolyte pad 24' are brought into contact with each other by removing the barrier film 9' to allow the electrolyte 24'a contained in the electrolyte pad 24' to participate in the supply and receipt of electrons among the sensitizing dye, the working electrode, and the counter electrode. A contact for connecting the working electrode to the ammeter is preferably ensured by a contact probe 28 in the sensor unit shown in Figs. 7 to 9 and by a contact probe 28' in the sensor unit shown in Figs. 10 to 12. This is true of the counter electrode.

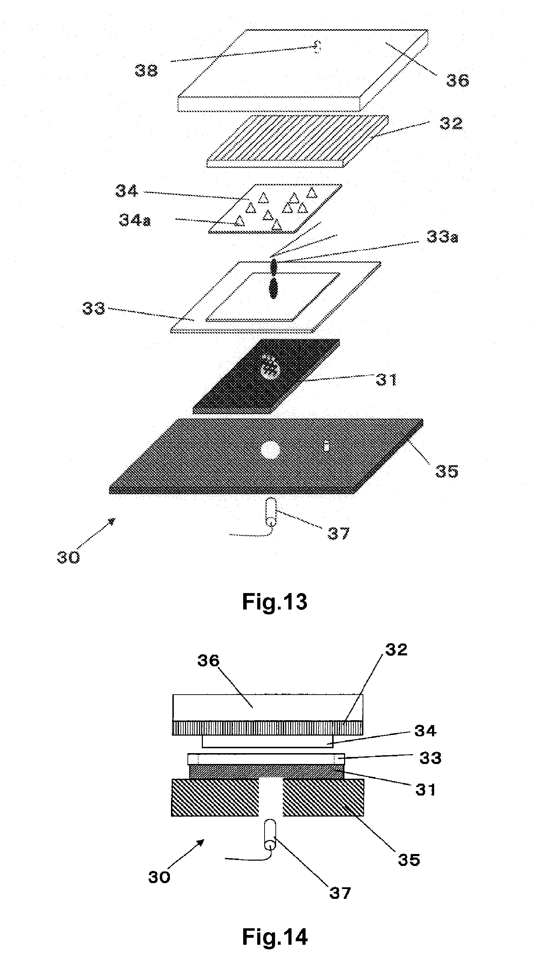

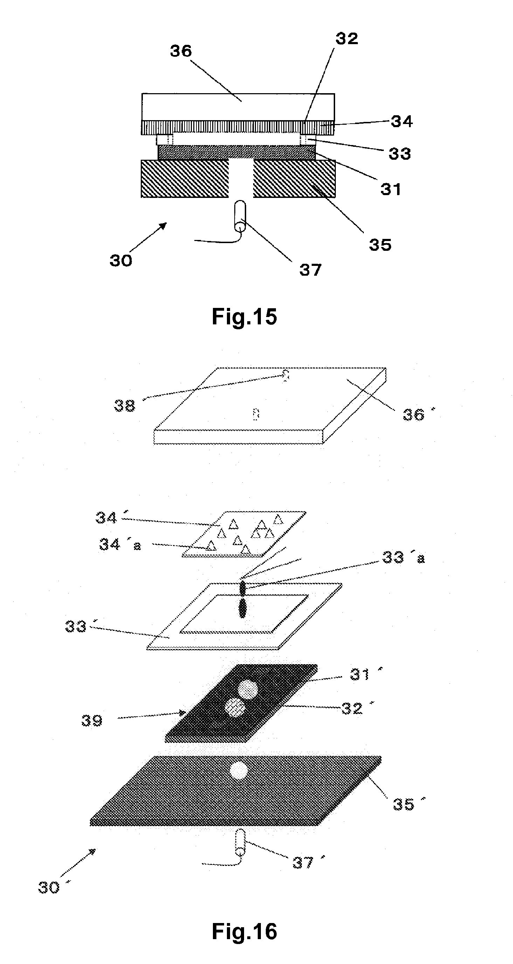

The sensor unit in the third embodiment of the present invention comprises a working electrode, a counter electrode, a spacer held between the working electrode and the counter electrode, and an electrolyte pad. In the specific reaction, a sample solution is present within an opening in the spacer in contact with the working electrode, and, in the detection of current, the sample solution within the spacer is interfused into the electrolyte pad. The electrolyte pad is brought into contact with the working electrode and the counter electrode to allow the electrolyte to participate in the supply and receipt of electrons among the sensitizing dye, the working electrode, and the counter electrode. That is, in the specific reaction, the working electrode is brought into contact with the sample solution within the spacer, and, after the completion of the step of the specific reaction, the electrolyte pad is brought into contact with the sample solution within the spacer to detect current. Figs. 13, 14, and 15 are respectively a typical exploded view of a sensor unit in the third embodiment, a cross-sectional view of the sensor unit in the specific reaction, and a cross-sectional view of the sensor unit in the detection of current. In Fig. 13, a sensor unit 30 basically comprises a working electrode 31, a counter electrode 32, a spacer 33, and an electrolyte pad 34 and further comprises a pressing member 35 that supports the working electrode 31. A counter member 36 is provided on the uppermost part of the sensor unit 30 to press downward the working electrode 31, the spacer 33, the electrolyte pad 34, and the counter electrode 32 stacked in that order on the pressing member 35 to bring them into close contact with each other. In the specific reaction, as shown in Fig. 14, the working electrode 31 and a sample solution 33a within the spacer 33 are brought into contact with each other. Although the sample solution 33a within the spacer 33 and the electrolyte pad 34 are provided away from each other, in the detection of current, as shown in Fig. 15, the sample solution 33a within the spacer 33 is interfused into the electrolyte pad 34, and, further, the electrolyte pad 34 is brought into contact with the working electrode 31 and the counter electrode 32 to allow an electrolyte 34a contained in the electrolyte pad 34 to participate in the supply and receipt of electrons among the sensitizing dye, the working electrode, and the counter electrode.

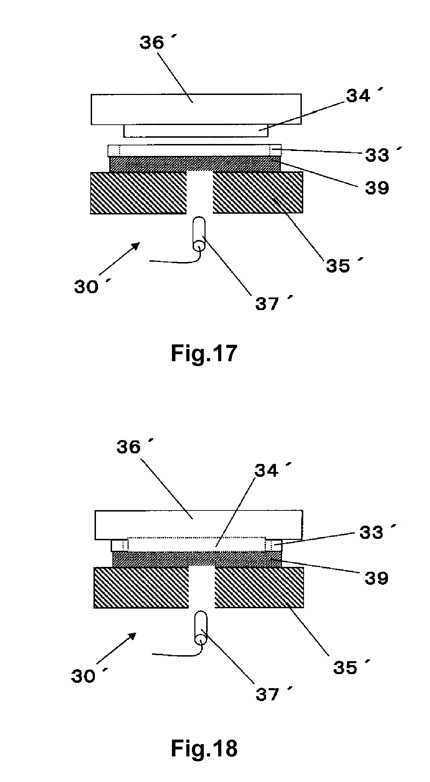

Figs. 16, 17, and 18 are respectively a typical exploded view of a sensor unit 30' using an electrode unit comprising a working electrode 31' and a counter electrode 32' patterned on the same plane in the sensor unit shown in Fig. 5, a cross-sectional view of the sensor unit 30' in the specific reaction, and a cross-sectional view of the sensor unit 30' in the detection of current, which are a variant of the sensor unit in the third embodiment. In the sensor unit 30' shown in Figs. 16 to 18, a counter member 36' is provided so as to face an electrode unit 39, and a spacer 33' and an electrolyte pad 34' are held between the electrode unit 39 and the counter member 35'. The sensor unit 30' further comprises a pressing member 35' that supports the electrode unit 39. That is, the electrode unit 39 is disposed on the pressing member 35' so that the analyte-immobilized side faces upward. In the specific reaction, as shown in Fig. 17, the electrode unit 39 is brought into contact with the sample solution 33'a within the spacer 33'. Although the sample solution 33'a within the spacer 33' and the electrolyte pad 34' are away from each other, in the detection of current, as shown in Fig. 6, the sample solution 33'a within the spacer 33' and the electrolyte pad 34' are brought into contact with each other to allow the electrolyte 34'a contained in the electrolyte pad 34' to participate in the supply and receipt of electrons among the sensitizing dye, the working electrode, and the counter electrode. A contact for connecting the working electrode to the ammeter is preferably ensured by a contact probe 38 in the sensor unit shown in Figs. 13 to 15 and by a contact probe 38' in the sensor unit shown in Figs. 16 to 18. This is true of the counter electrode.

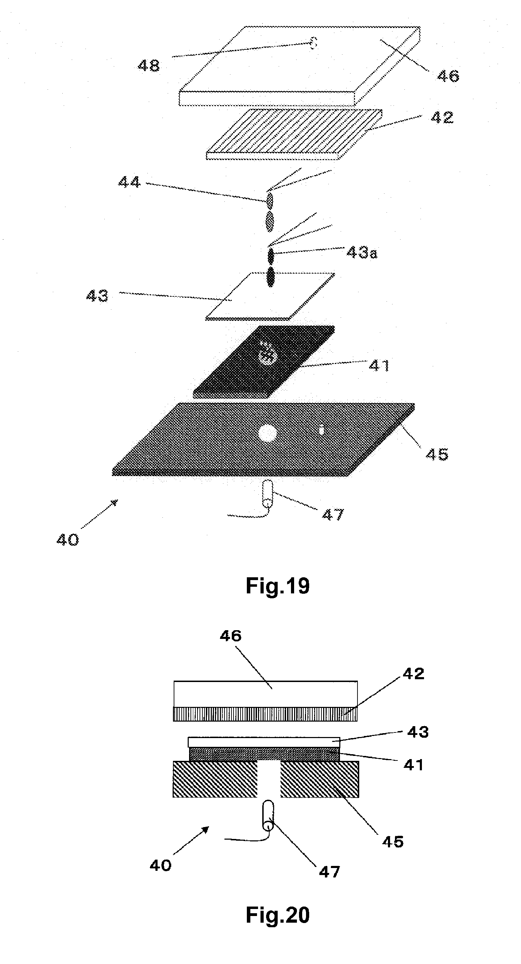

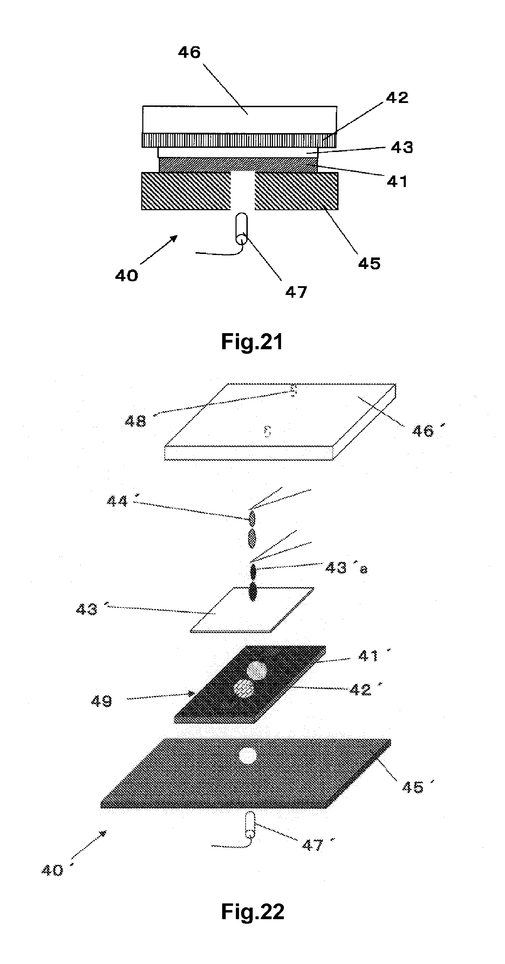

The sensor unit in the fourth embodiment of the present invention comprises a working electrode, a counter electrode, and a reaction pad held between the working electrode and the counter electrode. In the specific reaction, the working electrode comes into contact with the sample solution-added reaction pad, and, in the detection of current, an electrolyte solution is added to the reaction pad to allow the electrolyte to participate in the supply and receipt of electrons among the sensitizing dye, the working electrode, and the counter electrode. That is, in the specific reaction, the working electrode is brought into contact with the sample solution-added reaction pad, and, after the completion of the step of the specific reaction, the electrolyte solution is added to the reaction pad to detect current. Figs. 19, 20, and 21 are respectively a typical exploded view of a sensor unit in the fourth embodiment, a cross-sectional view of the sensor unit in the specific reaction, and a cross-sectional view of the sensor unit in the detection of current. In Fig. 19, a sensor unit 40 basically comprises a working electrode 41, a counter electrode 42, and a reaction pad 43 and further comprises a pressing member 45 that supports the working electrode 41. A counter member 46 is provided on the uppermost part of the sensor unit 40 to press downward the working electrode 41, the reaction pad 43, and the counter electrode 42 stacked in that order on the pressing member 45 to bring them into close contact with each other. In the specific reaction, as shown in Fig. 20, although the working electrode 41 and the reaction pad 43 to which a sample solution 43a has been added are brought into contact with each other, in the detection of current, as shown in Fig. 21, an electrolyte solution 44 is added to the reaction pad 43 to allow an electrolyte contained in the electrolyte solution to participate in the supply and receipt of electrons among the sensitizing dye, the working electrode, and the counter electrode.

In the sensor unit of the present invention, the order of components to be stacked is not limited to the embodiment shown in the drawing, and the components may be stacked in the reverse order of the order shown in Fig. 7 to turn the laminate upside down. In this case, a construction is adopted in which a counter member for supporting the counter electrode and a pressing member for pressing downward the counter electrode, the reaction pad, and the working electrode stacked in that order on the counter member to bring them into close contact with each other are provided. In the embodiment shown in the drawing, the members are horizontally arranged, but may be arranged in an upright state. Preferably, the pressing member 45 further comprises an opening or a light-transparent part that allows light for photoexcitation from the light source 47 to be passed thereinto.

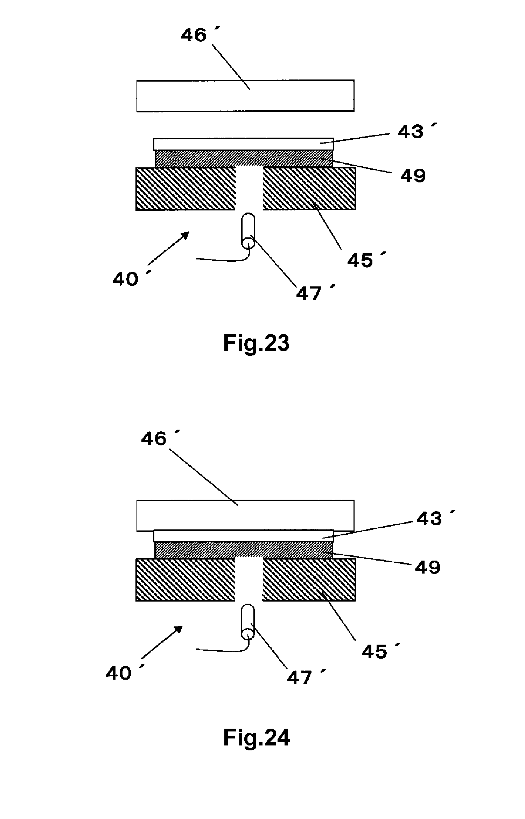

Figs. 22, 23, and 24 are respectively a typical exploded view of a sensor unit 40' using an electrode unit comprising a working electrode 41' and a counter electrode 42' patterned on the same plane in the sensor unit shown in Fig. 7, a cross-sectional view of the sensor unit 40' in the specific reaction, and a cross-sectional view of the sensor unit 40' in the detection of current, which are a variant of the sensor unit in the fourth embodiment. In the sensor unit 40' shown in Figs. 22 to 24, a counter member 46' is provided so as to face an electrode unit 49, and a reaction pad 43' is held between the electrode unit 49 and the counter member 46'. The sensor unit 40' further comprises a pressing member 45' that supports the electrode unit 49. That is, the electrode unit 49 is disposed on the pressing member 45' so that the analyte-immobilized side faces upward. In the specific reaction, as shown in Fig. 23, the electrode unit 49 is brought into contact with the reaction pad 43' to which the sample solution 43'a has been added. In the detection of current, as shown in Fig. 24, an electrolyte solution 44' is added to the reaction pad 43' to allow the electrolyte contained in the electrolyte solution 44' to participate in the supply and receipt of electrons among the sensitizing dye, the working electrode, and the counter electrode. A contact for connecting the working electrode to the ammeter is preferably ensured by a contact probe 48 in the sensor unit shown in Figs. 19 to 21 and by a contact probe 48' in the sensor unit shown in Figs. 22 to 24. This is true of the counter electrode.

In any of the first to fourth embodiments, a light source 17, 17', 27, 27', 37, 37', 47, or 47' for applying light to the working electrode and an ammeter (not shown) for measuring current which flows across the working electrode and the counter electrode can be further provided in the sensor unit 10, 10', 20, 20', 30, 30', 40, or 40' to construct a measuring device. Preferably, the ammeter can detect current on an nA level. In this construction, light from a light source is applied to the surface of the working electrode. In the sensor units 10, 10', 20, 20', 30, 30', 40, and 40' shown in Figs. 1 to 22, light from the light source 17, 17', 27, 27', 37, 37', 47, or 47' is applied through the back side of the working electrode 11, 21, 31, or 41 or the electrode unit 19, 29, 39, or 49, passes through the transparent working electrode 11, 21, 31, or 41 or the electrode unit 19, 29, 39, or 49, and reaches the surface of the working electrode 11, 21, 31, or 41 or the electrode unit 19, 29, 39, or 49. The value of the photocurrent generated by photoexcitation of a sensitizing dye by the light which has reached the working electrode can be detected with the ammeter. The working electrode and the counter electrode may be connected to the ammeter by any mean without particular limitation. For example, means such as direct connection of a lead wire or connection through a contact probe 18, 18', 28, 28', 38, 38', 48, or 48' as in the embodiment shown in the drawing may be adopted. In particular, for a working electrode which is attached/detached for each measurement, the use of a contact probe can advantageously facilitate the detachment/attachment of the working electrode.

In a preferred embodiment of the present invention, a measuring device comprising a combination of a plurality of sensor units can also be prepared. In this case, in the sensor unit, a plurality of light sources may be previously provided, and the measuring device may further comprise a mechanism that, in the application of light, switches the plurality of the light sources.

In a preferred embodiment of the present invention, an XY transfer mechanism (not shown) is mounted on the measuring device comprising the combination of the plurality of sensor units so that the light source and the sensor unit are relatively moved in the XY direction to allow light to be applied while the light source is scanned and moved in the XY direction on the working electrode. Thus, light can be applied to analyte-immobilized spots on the plurality of sensor units. In particular, in the sensor unit according to the present invention, since the supply of the electrolyte solution is unnecessary, the structure is simplified and, consequently, the sensor unit per se can easily be moved in the XY direction. In this case, preferably, the XY transfer mechanism is constructed so that the movement speed, movement path and the like can be designated by a software incorporated in a computer or the device.

In a preferred embodiment of the present invention, the value of current generated by light irradiation is measured with the ammeter, and the results are sent to a memory in the computer or the device and are successively stored as data. The photocurrent values thus stored in the memory can be displayed on a display monitor as numeral values or a time-dependent change graph representing real-time data. Current values in light non-irradiation and in the light irradiation are read from proper data points based on the data thus obtained, and the difference in current value between the light non-irradiation and the light irradiation may be used to quantitatively determine the concentration of substances in the sample. Further, a process from reading of the data to the quantitative determination can also be automatically processed on the software.

### Special detection of analyte using photocurrent

As described above, the method and sensor unit according to the present invention are used in the specific detection of an analyte using photocurrent generated by photoexcitation of a sensitizing dye. The method for specifically detecting an analyte using photocurrent generated by photoexcitation of the sensitizing dye will be specifically described.

The method for specifically detecting an analyte using photocurrent in a preferred embodiment of the present invention comprises bringing a working electrode having on its surface a probe substance, to which an analyte can be specifically bound directly or indirectly, into contact with a sample solution holding member such as a reaction pad or a spacer, adding a sample solution to the sample solution holding member, then applying light to the working electrode with the sensitizing dye immobilized thereon through specific binding to the probe substance to photoexcite the sensitizing dye, and detecting photocurrent that flows, across the working electrode and the counter electrode in contact with the pad, attributable to the transfer of electrons from the photoexcited sensitizing dye to the working electrode.

According to this method, at the outset, a sample solution containing at least an analyte and a solvent and a working electrode are provided. The solvent is a liquid that can dissolve or disperse the analyte, the sensitizing dye, and the electrolyte and is water and/or an aqueous solvent, preferably water. The working electrode used in the present invention is an electrode that has on its surface a probe substance capable of specifically binding to the analyte directly or indirectly. That is, the probe substance may be a substance that can specifically bind directly to the analyte, as well as a substance that can specifically bind to a conjugate obtained by binding an analyte specifically to a mediator substance such as a receptor protein molecule. A sample solution is brought into contact with the working electrode in the copresence of a sensitizing dye to specifically bind the analyte to the probe substance directly or indirectly, and the sensitizing dye is immobilized onto the working electrode through this binding. The sensitizing dye is a substance that can release electrons to the working electrode in response to the photoexcitation. When the method for specifically detecting the analyte is a sandwich method, the probe substance is a primary antibody and the sensitizing dye is labeled on a secondary antibody. When the method for specifically detecting the analyte is a receptor binding assay, the probe substance is peptide, protein or DNA and the sensitizing dye is labeled on a receptor protein. When the method for specifically detecting the analyte is a competitive method, the sensitizing dye is labeled on a second analyte that can be specifically bound to the probe substance. The sensitizing dye-labeled substance may be contained in any of the sample solution and the reaction pad.

The working electrode is brought into contact with a sample solution holding member such as a reaction pad or a spacer. A sample solution is added to the sample solution holding member. Thereafter, light is applied to the working electrode with the sensitizing dye immobilized thereon through specific binding to the probe substance to photoexcite the sensitizing dye. As a result, the transfer of electrons occurs from the photoexcited sensitizing dye to the electron-receptive substance. The analyte can be detected with high sensitivity and accuracy by detecting photocurrent that flows, across the working electrode and the counter electrode, attributable to the transfer of electrons. Further, since the detected current is highly correlated with the concentration of the analyte in the sample solution, the analyte can be quantitatively measured based on the measured amount of current and the measured electrical quantity.

Preferably, the electrolyte used in the present invention is not present in the sample solution within a sample solution holding member such as a reaction pad or a spacer in the specific reaction. When the electrolyte is present in the specific reaction, the specific reaction with the probe substance is less likely to take place, leading to lowered detection sensitivity. Accordingly, preferably, the electrolyte is present in the reaction pad or the electrolyte pad only in the detection of current.

### Analyte and probe substance

In the present invention, the analyte may be any substance without particular limitation as long as the substance can specifically bind to the probe substance. In the method according to the present invention, when the probe substance that can specifically bind to the analyte directly or indirectly is supported on the surface of the working electrode, the analyte can be detected by specifically binding the analyte to the probe substance directly or indirectly.

That is, in the present invention, the analyte and the probe substance selected may be such that they can specifically bind to each other. Specifically, in a preferred embodiment of the present invention, a substance having a specific binding capability is used as the analyte, and a substance capable of specifically binding to the analyte is supported as the probe substance onto the working electrode. This makes it possible to specifically bind the analyte directly onto the working electrode and to detect the analyte. A preferred example of a combination of an analyte and a probe substance in an embodiment wherein the analyte is specifically directly bound is a combination of an antigen with an antibody. On the other hand, an example using a sandwich method which will be described later, or an example using receptor binding assay may be mentioned as a preferred embodiment of a combination of an analyte with a probe substance in an embodiment wherein the analyte is indirectly specifically bound to the probe substance.

### Measuring method

In the measuring method using the sensor unit according to the present invention, at the outset, a probe substance to which an analyte can be specifically bound directly or indirectly is immobilized on the surface of the working electrode. The working electrode onto which the probe substance capable of specifically binding to the analyte directly or indirectly is immobilized is brought into contact with a sample solution holding member such as a reaction pad or a spacer. After a sample solution containing an analyte is spot-stuck, a sensitizing dye is immobilized onto the working electrode through direct or indirect specific binding.

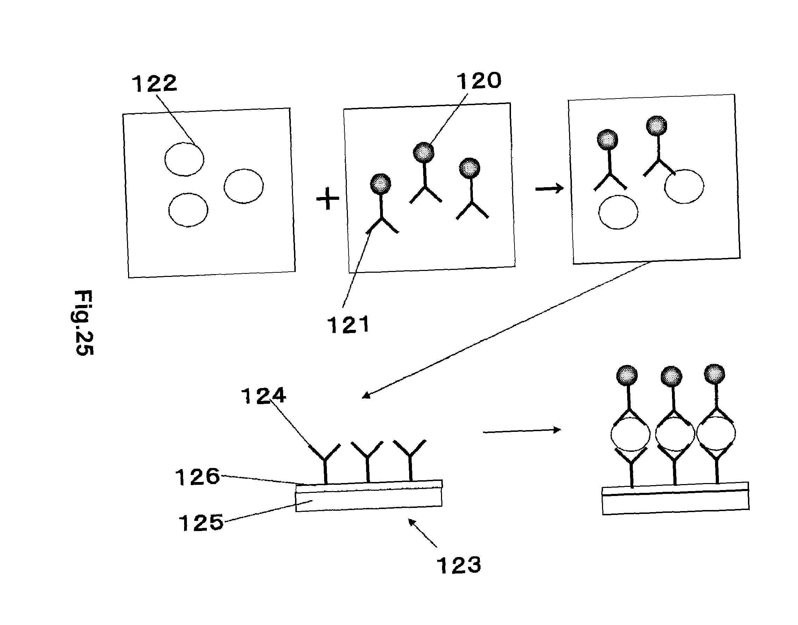

In a preferred embodiment of the present invention, a method may be adopted in which an antigen is used as the analyte and an immunoassay, particularly a sandwich method, is used. In this case, a primary antibody is used as the probe substance, an antigen as the analyte is specifically bound to the primary antibody, and a secondary antibody labeled with the sensitizing dye is immobilized onto the working electrode through specific binding to the antigen. The step of immobilizing the antigen as the analyte and the secondary antibody labeled with the sensitizing dye to the primary antibody as the probe substance in this embodiment is shown in Fig. 25. As shown in Fig. 25, a secondary antibody 121 labeled with a sensitizing dye 120 is specifically bound to an antibody 123 in the presence of an antigen 122 as the analyte.

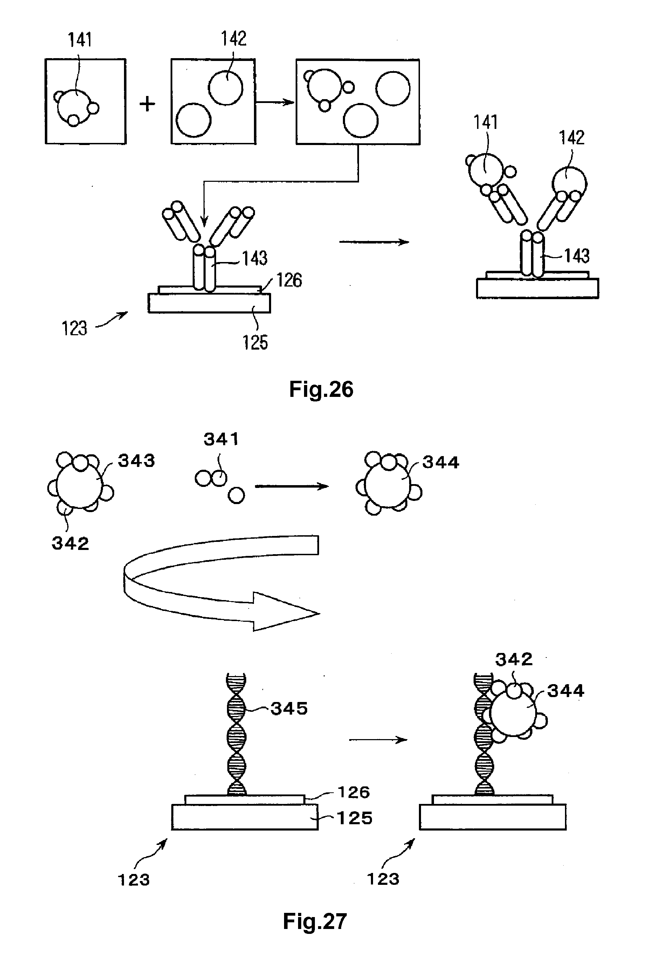

In another preferred embodiment of the present invention, a competitive immunoassay using an antigen as the analyte may be adopted. In this case, an antigen as the analyte and an antigen as the second analyte having the same binding capability as the analyte and labeled with a sensitizing dye are competitively bound to the probe substance which is an antibody, whereby the sensitizing dye is immobilized onto the working electrode. The step of immobilizing the antigen as the analyte and the antigen as the second analyte labeled with the sensitizing dye onto the antibody as the probe substance in this embodiment is shown in Fig. 26. As shown in Fig. 26, an antigen 141 as the second analyte labeled with the sensitizing dye and an antigen 142 as the analyte not labeled with the dye are competitively specifically bound to an antibody 143.

In the present invention, the specific binding between the analyte and the probe substance may be indirect binding. In a preferred embodiment of the present invention, receptor bound assay may be adopted. In this case, a receptor protein labeled with a sensitizing dye capable of specifically binding to a ligand as the analyte is allowed to coexist, and the sensitizing dye is immobilized onto the working electrode with a nucleic acid (or peptide, protein or the like) immobilized thereonto in the presence of a ligand. The step of immobilizing the ligand as the analyte onto the working electrode in this embodiment is shown in Fig. 27. As shown in Fig. 27, a ligand 341 as the analyte is first specifically bound to a receptor protein 343 labeled with a sensitizing dye 342. A receptor protein 344 to which the ligand has been bound is specifically bound to a double stranded nucleic acid 345 as the probe substance.

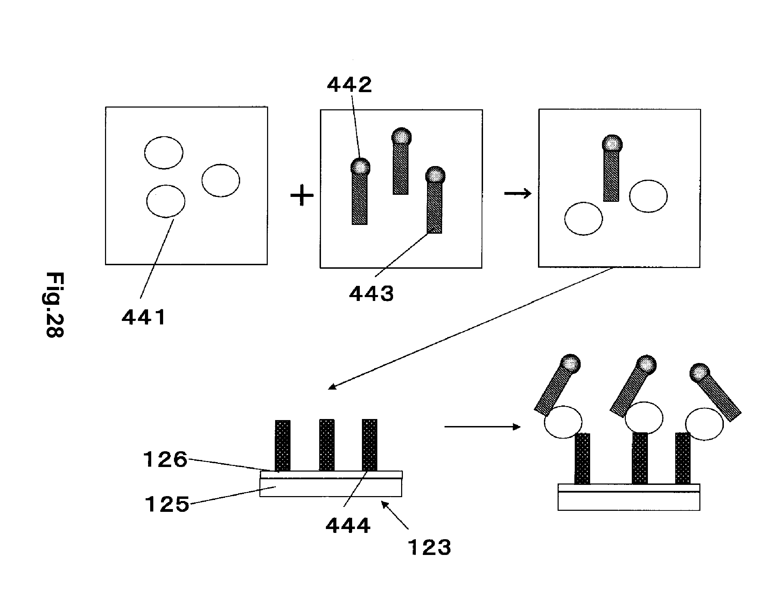

In another preferred embodiment of the present invention, immunoassay by an open sandwich method may also be adopted. In this case, an L chain or an H chain in an antibody labeled with a sensitizing dye capable of specifically binding to an antigen as an analyte is allowed to exist. The sensitizing dye is immobilized onto a working electrode with an H chain or an L chain in an antibody as a probe substance immobilized thereonto in the presence of an analyte. The step of immobilizing the antigen as the analyte onto the working electrode in this embodiment is shown in Fig. 28. As shown in Fig. 28, an antigen 441 as the analyte is specifically bound to an H chain 444 (or an L chain) in the antibody as the probe substance in the presence of an L chain 443 (or an H chain) in the antibody labeled with a sensitizing dye 442.

The working electrode with the analyte together with the sensitizing dye immobilized thereonto is irradiated with light in the presence of an electrolyte to photoexcite the sensitizing dye, and photocurrent which flows, across the working electrode and the counter electrode in contact with the electrolyte-containing pad, attributable to the transfer of electrons from the photoexcited sensitizing dye to the working electrode is detected with an ammeter.

Photocurrent which flows in the system upon light irradiation is measured with an ammeter. Thus, the analyte can be detected. In this case, the current value reflects the amount of the sensitizing dye trapped on the working electrode. For example, when the analyte is an antigen, the amount of the antibody bound specifically to the antibody is reflected in the current value. Accordingly, the analyte can be quantitatively determined from the current value. Thus, in a preferred embodiment of the present invention, the ammeter further comprises means that calculates the concentration of the analyte in the sample solution from the measured amount of current or electrical quantity.

In a preferred embodiment of the present invention, in the step of detecting photocurrent, a current value is measured, and the concentration of the analyte in the sample solution can be calculated from the current value or electrical quantity thus obtained. The calculation of the analyte concentration can be carried out by comparing a previously prepared calibration curve for the analyte concentration and the current value or electrical quantity with the obtained current value or electrical quantity. In the method according to the present invention, the amount of the sensitizing dye trapped on the working electrode is reflected in the current value. Accordingly, an accurate current value corresponding to the concentration of the analyte can be obtained, and, thus, the method is suitable for quantitative determination.

In a preferred embodiment of the present invention, when the competitive method is adopted, an antigen or single stranded nucleic acid as an analyte and an antigen or single stranded nucleic acid labeled with a sensitizing dye can be competitively bound specifically to the probe substance to provide a correlation between the detected current value and the concentration of the antigen or single stranded nucleic acid labeled with the sensitizing dye. That is, as the number of analytes not labeled with the sensitizing dye increases, the number of competitive substances specifically bound to the probe substance decreases. Accordingly, a calibration curve can be obtained in which, as the concentration of the analyte not labeled with the dye increases, the detected current value decreases. Therefore, the detection and quantitative determination of the analyte not labeled with the sensitizing dye are possible.

### Sensitizing dye

In the present invention, in order to detect the presence of an analyte through photocurrent, an analyte is specifically bound to a probe substance directly or indirectly in the copresence of a sensitizing dye to immobilize the sensitizing dye onto a working electrode by the binding. To this end, in the present invention, preferably, as shown in Figs. 25 to 28, the sensitizing dye is labeled on an affinity substance such as a primary antibody, an antigen, a ligand, a receptor protein, a single stranded nucleic acid, an H chain in an antibody, or an L chain in an antibody.

The sensitizing dye used in the present invention is a substance that can release electrons to the working electrode in response to photoexcitation. The sensitizing dye may be one that can be transited to a photoexcited state upon exposure to light from a light source and can take, from the excited state, an electron state which allows electrons to be injected into the working electrode. Accordingly, the sensitizing dye may be one that can take the electron state between the sensitizing dye and the working electrode, particularly an electron-receptive layer. Therefore, a variety of sensitizing dyes are usable, and there is no need to use expensive dyes.

Specific examples of sensitizing dyes include metal complexes and organic dyes. Examples of preferred metal complexes include metal phthalocyanines such as copper phthalocyanine and titanylphthalocyanine; chlorophyll or its derivatives; hemin; and ruthenium, osmium, iron, and zinc complexes (for example, cis-dicyanate-bis(2,2'-bipyridyl-4,4'-dicarboxylate)ruthenium(II)) described in Japanese Patent Application Laid-Open No. 220380/1989 and Japanese Translation of PCT Publication No. 504023/1993. Examples of preferred organic dyes include metal-free phthalocyanine, 9-phenylxanthene dyes, cyanine dyes, metallocyanine dyes, xanthene dyes, triphenylmethane dyes, acridine dyes, oxazine dyes, coumarin dyes, merocyanine dyes, rhodacyanine dyes, polymethine dyes, and indigo dyes.

### Working electrode and manufacture thereof

The working electrode used in the present invention is an electrode that has on its surface the above probe substance and can receive electrons released from a sensitizing dye, immobilized onto the working electrode through the probe substance, in response to photoexcitation. Accordingly, for the working electrode, various constructions and materials may be used without limitation as long as electron transfer takes place between the working electrode and the sensitizing dye.

In a preferred embodiment of the present invention, the working electrode comprises an electron-receptive layer comprising an electron-receptive substance capable of receiving electrons released in response to photoexcitation of the sensitizing dye, and comprises a probe substance provided on the surface of the electron-receptive layer. In a preferred embodiment of the present invention, the working electrode further comprises an electroconductive substrate. Preferably, the electron-receptive layer is provided on the electroconductive substrate. The electrode in this embodiment is shown in Figs. 25 to 28. A working electrode 123 shown in Figs. 25 to 28 comprises an electroconductive substrate 125 and an electron-receptive layer 126 that is provided on the electroconductive substrate and comprises an electron-receptive substance. A probe substance is supported on the surface of the electron-receptive layer 126.

In the present invention, the electron-receptive layer comprises an electron-receptive substance capable of receiving electrons released from a sensitizing dye immobilized through a probe substance in response to photoexcitation. That is, the electron-receptive substance may be a substance that can take such an energy level that electrons from the photoexcited labeled dye can be injected thereinto. The energy level (A) on which electrons from the photoexcited labeled dye can be injected means, for example, a conduction band (CB) when a semiconductor is used as an electron-receptive substance; a Fermi level when a metal is used as the electron-receptive substance; and a lowest unoccupied molecular orbital (LUMO) when an organic substance or a molecular inorganic substance such as C₆₀ is used as the electron-receptive substance. That is, the electron-receptive substance used in the present invention may be one that has a level A that is baser than the energy level of the LUMO of the sensitizing dye, in other words, an energy level lower than that of the LUMO of the sensitizing dye.

Examples of preferred electron-receptive substances include element semiconductors of silicon, germanium or the like; oxide semiconductors of titanium, tin, zinc, iron, tungsten, zirconium, hafnium, strontium, indium, cerium, yttrium, lanthanum, vanadium, niobium, tantalum or the like; perovskite semiconductors of strontium titanate, calcium titanate, sodium titanate, barium titanate, potassium niobate and the like; sulfide semiconductors of cadmium, zinc, lead, silver, stibium and bismuth; selenide semiconductors of cadmium or lead; a telluride semiconductor of cadmium; phosphide semiconductors of zinc, gallium, indium, cadmium or the like; compound semiconductors of gallium arsenide, copper-indium-selenide, or copper-indium-sulfide; metals such as gold, platinum, silver, copper, aluminum, rhodium, indium, and nickel; organic polymers such as polythiophene, polyaniline, polyacetylene, and polypyrrole; and molecular inorganic substances such as C₆₀ and C₇₀, more preferably silicon, TiO₂, SnO₂, Fe₂O₃, WO₃, ZnO, Nb₂O₅, strontium titanate, indium oxide, CdS, ZnS, PbS, Bi₂S₃, CdSe, CdTe, GaP, InP, GaAs, CuInS₂, CuInSe, and C₆₀, further preferably TiO₂, ZnO, SnO₂, Fe₂O₃, WO₃, Nb₂O₅, strontium titanate, CdS, PbS, CdSe, InP, GaAs, CuInS₂, and CuInSe₂, most preferably TiO₂. The above semiconductors may be either an intrinsic semiconductor or an impurity semiconductor.

In a preferred embodiment of the present invention, the electron-receptive substance is a semiconductor, more preferably an oxide semiconductor, still more preferably a metal oxide semiconductor, most preferably an n-type metal oxide semiconductor. In this embodiment, electrons can be efficiently taken out from the dye by taking advantage of a bandgap of the semiconductor. Further, a working electrode having a large surface area can be prepared using a semiconductor having a porous structure or a structure having a concave and convex shape on its surface, and, in this case, the amount of the probe immobilized can be increased.

In a preferred embodiment of the present invention, the potential of the conductive band of the semiconductor is lower than the potential of LUMO of the sensitizing dye, more preferably a potential that meets a relationship of LUMO of sensitizing dye > conductive band of semiconductor > oxidation-reduction potential of electrolyte > HOMO of sensitizing dye. This relationship can allow electrons to be efficiently taken out.

In a preferred embodiment of the present invention, when the electron-receptive layer is formed of a semiconductor, the layer surface can be cationized. The cationization can allow the probe substance (DNA, protein or the like) to be highly efficiently adsorbed onto the electron-receptive layer. The cationization can be carried out, for example, by allowing a silane coupling agent such as aminosilane, a cationic polymer, or a quaternary ammonium compound to act on the surface of the electron-receptive layer.

In another preferred embodiment of the present invention, indium-tin oxide (ITO) or fluorine-doped tin oxide (FTO) may be used as the electron-receptive substance. Since ITO and FTO function not only as an electron-receptive layer but also as an electroconductive substrate, the use of these substances allows the electron-receptive layer alone to function as a working electrode without use of an electroconductive substrate.

When a semiconductor or a metal is used as an electron-receptive substance, the semiconductor or the metal may be either a single crystal or polycrystal, preferably a polycrystal, more preferably a porous crystal rather than a dense polycrystal. Therefore, the semiconductor or the metal has an increased specific surface area, can adsorb a larger amount of the analyte and the sensitizing dye, and can realize the detection of the analyte with higher sensitivity and accuracy. In a preferred embodiment of the present invention, the electron-receptive layer is porous, and the diameter of each pore is preferably 3 to 1000 nm, more preferably 10 to 100 nm.

In a preferred embodiment of the present invention, the surface area in such a state that the electron-receptive layer is provided on the electroconductive substrate is preferably 10 or more times, more preferably 100 or more times, that of the projected area. The upper limit of the surface area is not particularly limited, but will be generally approximately 1000 times. The diameter of fine particles of the electron-receptive substance constituting the electron-receptive layer is preferably 5 to 200 nm, more preferably 8 to 100 nm, still more preferably 20 to 60 nm, in terms of average particle diameter of primary particles using an equivalent circular diameter of the projected area. The average particle diameter of fine particles (secondary particles) of the electron-receptive substance in the dispersion is preferably 0.01 to 100 µm. From the viewpoint of scattering incident light to improve the percentage light trapping, fine particles of the electron-receptive substance having a large particle size of, for example, about 300 nm are additionally used in combination to form the electron-receptive layer.

In a preferred embodiment of the present invention, the working electrode further comprises an electroconductive substrate, and the electron-receptive layer is provided on the electroconductive substrate. The electroconductive substrate usable in the present invention may be not only one that has a support which as such has electroconductive properties, for example, metals such as titanium, but also one that has a electroconductive material layer provided on the surface of a glass or plastic support. When the electroconductive substrate having the electroconductive material layer is used, the electron-receptive layer is provided on the electroconductive layer. Examples of electroconductive materials constituting the electroconductive material layer include metals such as platinum, gold, silver, copper, aluminum, rhodium, and indium; electroconductive ceramics such as carbon, carbide and nitride; and electroconductive metallic oxides such as indium-tin oxide, fluorine-doped tin oxide, antimony-doped tin oxide, gallium-doped zinc oxide, and aluminum-doped zinc oxide, preferably indium-tin oxide (ITO) and fluorine-doped tin oxide (FTO). As described above, however, when the electron-receptive layer per se functions as an electroconductive substrate, the electroconductive substrate may be omitted. Further, in the present invention, the electroconductive substrate is not limited as long as electroconductive properties can be ensured, and includes thin film-shaped or spot-shaped electroconductive material layers that as such does not have strength high enough to be used as the support.

In a preferred embodiment of the present invention, the electroconductive substrate is substantially transparent, specifically preferably has a light transmittance of not less than 10%, more preferably not less than 50%, still more preferably not less than 70%. Further, in a preferred embodiment of the present invention, the electroconductive material layer has a thickness of 0.02 to 10 µm. Furthermore, in a preferred embodiment of the present invention, the electroconductive substrate has a surface resistivity of not more than 100 Ω/cm², more preferably not more than 40 Ω/cm². The lower limit of the surface resistivity of the electroconductive substrate is not particularly limited, but would be generally approximately 0.1 Ω/cm².

Examples of preferred methods for forming the electron-receptive layer on the electroconductive substrate include a method in which a dispersion or a colloidal solution of the electron-receptive substance is coated onto an electroconductive support; a method in which a precursor of fine particles of a semiconductor is coated onto an electroconductive support and is hydrolyzed by moisture in the air to form a film of fine particles (a sol-gel process); sputtering; CVD; PVD; and vapor-deposition. Examples of methods for preparing a dispersion of fine particles of a semiconductor as the electron-receptive substance include, in addition to the above sol-gel process, a method in which the particles are ground in a mortar; a method in which the particles are dispersed while grinding with a mill; or a method in which fine-particles are precipitated in a solvent during the synthesis of a semiconductor and as such are then used. Examples of dispersion media usable herein include water or various organic solvents (for example, methanol, ethanol, isopropyl alcohol, dichloromethane, acetone, acetonitrile, and ethyl acetate). In the dispersion process, if necessary, polymers, surfactants, acids, chelating agents or the like may be used as a dispersing assistant.

Examples of preferred methods for coating a dispersion or colloidal solution of the electron-receptive substance include application methods such as roller coating and dipping; metering methods such as air knife coating and blade coating; wire-bar coating disclosed in Japanese Patent Publication No. 4589/1983, slide hopper coating, extrusion coating, curtain coating, spin coating, and spray coating described in US patent No. 2681294, US patent No. 2761419, US patent No. 2761791 and the like, as a method that can perform application and metering at the same part.

In a preferred embodiment of the present invention, when the electron-receptive layer is formed of fine particles of a semiconductor, the thickness of the electron-receptive layer is preferably 0.1 to 200 µm, more preferably 0.1 to 100 µm, further preferably 1 to 30 µm, most preferably 2 to 25 µm. When the thickness of the electron-receptive layer is in the above-defined range, the amount of the probe substance and the immobilized sensitizing dye per unit projection area can be increased to increase the amount of photocurrent and, at the same time, the loss of generated electrons by charge recombination can also be reduced. The coverage of the fine particles of the semiconductor per m² of the electroconductive substrate is preferably 0.5 to 400 g, more preferably 5 to 100 g.

In a preferred embodiment of the present invention, when the electron-receptive substance comprises indium-tin oxide (ITO) or fluorine-doped tin oxide (FTO), the thickness of the electron-receptive layer is preferably not less than 1 nm, more preferably 10 nm to 1 µm.

In a preferred embodiment of the present invention, heat treatment is carried out after the fine particles of the semiconductor are coated onto the electroconductive substrate. The adoption of the heat treatment can allow particles to come into electrical contact with one another and, at the same time, can improve the strength of the coating film and the adhesion to the support. The heat treatment temperature is preferably 40 to 700°C, more preferably 100 to 600°C. The heat treatment time is preferably approximately 10 min to 10 hr.

In another preferred embodiment of the present invention, when an electroconductive substrate formed of a material having a low melting point or softening point, for example, a polymer film is used, film formation is carried out by a method that does not use high-temperature treatment, from the viewpoint of preventing a deterioration by heat. Examples of such film forming methods include pressing, low-temperature heating, electron-beam irradiation, microwave irradiation, electrophoresis, sputtering, CVD, PVD, and vapor deposition.

The probe substance is supported on the surface of the electron-receptive layer in the working electrode thus obtained. The probe substance may be supported on the working electrode by a conventional well-known method. In a preferred embodiment of the present invention, when a single stranded nucleic acid is used as the probe substance, a method may be adopted in which an oxide layer is formed on the surface of the working electrode and the nucleic acid probe and the working electrode are bound to each other through the oxide layer. In this case, the nucleic acid probe can be immobilized onto the working electrode by introducing a functional group into the end of the nucleic acid. The nucleic acid probe into which the functional group has been introduced as such can be immobilized onto a carrier by an immobilization reaction. The function group can be introduced into the end of the nucleic acid by an enzymatic reaction or a DNA synthesizer. Enzymes usable in the enzymatic reaction include, for example, terminal deoxynucleotidyl transferase, polyA polymerase, polynucleotide kinase, DNA polymerase, polynucleotide adenyltransferase, and RNA ligase. The functional group may also be introduced by a polymerase chain reaction method (a PCR method), a nick translation method, or a random primer method. The functional group may be introduced into any part of the nucleic acid, such as 3'-end, 5'-end or a random position.

In a preferred embodiment of the present invention, functional groups suitable for immobilization of the probe substance onto the working electrode include amines, carboxylic acids, thiol group, hydroxyl group, and phosphoric acid. Further, in a preferred embodiment of the present invention, in order to strongly immobilize the probe substance onto the working electrode, a material that can perform crosslinking between the working electrode and the probe substance may also be used. Examples of preferred crosslinking materials include coupling agents such as silane coupling agents and titanate coupling agents and electroconductive polymers such as polythiophene, polyacetylene, polypyrrole, and polyaniline.

In a preferred embodiment of the present invention, the immobilization of the probe substance can also be efficiently carried out by a simpler method called physical adsorption. The physical adsorption of the probe substance onto the electrode surface can be carried out, for example, as follows. The electrode surface is first cleaned with distilled water and an alcohol using an ultrasonic cleaner. Thereafter, the electrode is inserted into a buffer solution containing a probe substance to adsorb the probe substance onto the surface of the carrier.

Further, the addition of a blocking agent after supporting of the probe substance can suppress nonspecific adsorption. The blocking agent usable herein is not limited as long as the substance can fill sites of the electron-receptive layer surface with no probe substance supported thereon and can be adsorbed onto the electron-receptive substance, for example, by chemical adsorption or physical adsorption. Preferably, the blocking agent is a substance containing a functional group that can be adsorbed through a chemical bond. Examples of blocking agents include functional group-containing substances such as a carboxylic acid group, a phosphate group, a sulfonate group, a hydroxyl group, an amino group, a pyridyl group, or amide.

### Counter electrode

The counter electrode used in the present invention is not particularly limited as long as a current flows across the counter electrode and the working electrode when the counter electrode is brought into contact with an electrolyte solution. Glass, plastic, ceramics and the like on which a metal or conductive oxide has been vapor-deposited may be used as the counter electrode. Alternatively, the counter electrode may be prepared by forming a metallic thin film having a thickness of not more than 5 µm, preferably 3 nm to 3 µm, for example, by vapor deposition or sputtering. Examples of preferred materials usable for the counter electrode include platinum, gold, palladium, nickel, carbon, electroconductive polymers such as polythiophene, electroconductive ceramic such as oxide, carbide and nitride, more preferably platinum and carbon, most preferably platinum. A thin film can be formed from these materials in the same manner as in the method for electron-receptive layer formation.

### Electrode unit

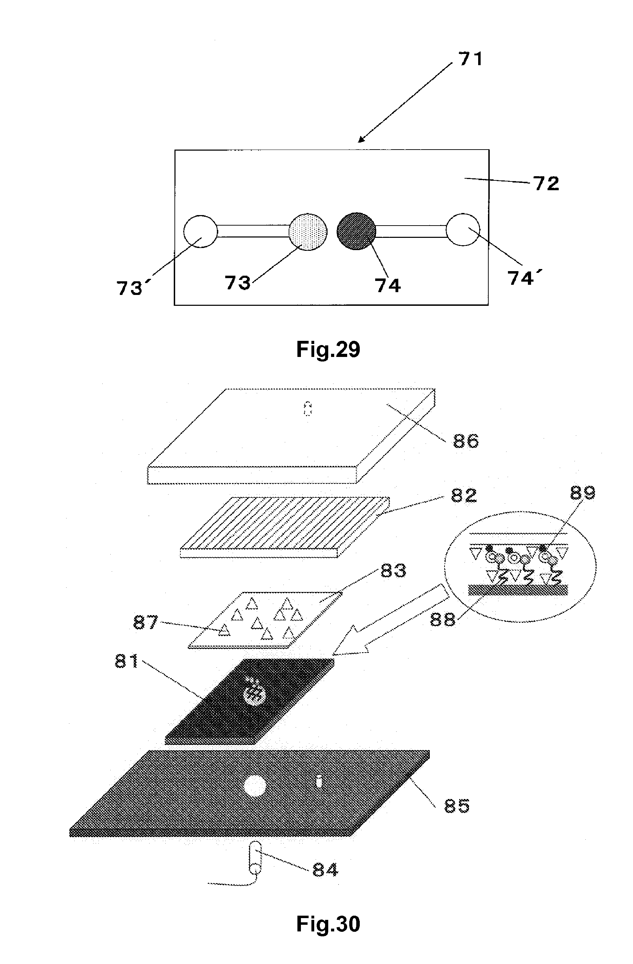

In a preferred embodiment of the present invention, an electrode unit comprising a working electrode and a counter electrode patterned on the same plane may be used. A preferred electrode unit comprises an insulating substrate, a working electrode that is locally provided on the insulating substrate and has an electron-receptive layer containing an electron-receptive substance capable of receiving electrons released from the sensitizing dye in response to photoexcitation, and a counter electrode provided away from the working electrode on the same plane as the working electrode on the insulating substrate. An embodiment of this electrode unit is shown in Fig. 29. An electrode unit 71 shown in Fig. 29 comprises an insulating substrate 72, a working electrode 73, and a counter electrode 74. The insulating substrate 72 is a substrate that has insulating properties high enough to prevent shortcircuiting between the working electrode 72 and the counter electrode 73. The working electrode 73 is locally provided on the insulating substrate 72 and comprises an electron-receptive layer containing an electron-receptive substance that can receive electrons released from the sensitizing dye in response to the photoexcitation. The counter electrode 74 is provided away from the working electrode 73 on the same plane as the working electrode 73 on the insulating substrate 72. Lead wires 73' and 74' are provided to extend from each of the working electrode 73 and the counter electrode 74, respectively.

Thus, the electrode unit is an integrated electrode member comprising a working electrode and a counter electrode on the same plane. The use of the electrode unit can realize a significant increase in degree of freedom in design and material selection of the sensor unit, contributing to significantly improved productivity, performance, and usability of the sensor unit. That is, since the electrode unit according to the present invention is an integrated electrode member and has no need to provide two electrodes so as to face each other, a construction in which the light source faces the surface of the electrode unit can easily be adopted. Consequently, the working electrode can be constructed using transparent materials, as well as opaque materials such as ceramics and plastics. This leads to an increase in the degree of freedom in the selection of electrode materials. Direct application of light from the light source to the surface of the working electrode can eliminate the loss of light attributable to the permeability of the transparent electrode material caused when the light is applied from the backside of the electrode. Accordingly, measurement with higher accuracy can be expected. Further, the electrode unit according to the present invention is an integrated electrode member, and, thus, the working electrode, the counter electrode, and the lead wire can be formed by a conductive patterning in one process, contributing to improved productivity of the electrode. Further, electroconductive properties are not required of the material provided opposite to the electrode unit, and, thus, widely used materials such as transparent plastics and glass can be used, contributing to improved productivity of the cell.

### EXAMPLES

The present invention is further illustrated by the following Examples that are not intended as a limitation of the invention.

### Example 1: Specific detection of protein with different electrolyte media

### (1) Preparation of working electrode with biotin-labeled DNA and ssDNA immobilized thereonto

A fluorine-doped tin oxide (F-SnO₂: FTO) coated glass (manufactured by Al Special Glass Company, U film, sheet resistance: 12 Ω/□, and shape: 50 mm × 26 mm) was provided as a glass substrate for a working electrode. This glass substrate was ultrasonically cleaned in acetone for 15 min and subsequently in ultrapure water for 15 min to remove contaminants and residual organic matter. The glass substrate was shaken in a 5 M aqueous sodium hydroxide for 15 min. Thereafter, shaking of the glass substrate in ultrapure water for 5 min was repeated three times while replacing water with fresh water for each shaking to remove sodium hydroxide. The glass substrate was taken out, and air was blown against the glass substrate to blow away the residual water. The glass substrate was then immersed in anhydrous methanol for dehydration.

3-Aminopropyltrimethoxysilane (APTMS) was added to a solvent composed of 95% methanol and 5% ultrapure water to bring the APTMS concentration to 2% by volume, and the mixture was stirred at room temperature for 5 min to prepare a solution for coupling treatment. The above glass substrate was immersed in the solution for coupling treatment, and was then slowly shaken for 15 min. The glass substrate was then taken out and was shaken approximately 10 times in methanol to remove excess solution for coupling treatment. This procedure was repeated three times while replacing methanol with fresh methanol for each time. Thereafter, the glass substrate was held at 110°C for 30 min to bind the coupling agent to the glass substrate. The glass substrate was cooled at room temperature, and a pressure-sensitive adhesive seal (thickness: 0.5 mm) having openings with a diameter of 3 mm was placed on and brought into close contact with the glass substrate. Subsequently, biotin-labeled ssDNA (25 mer), of which the concentration had been adjusted to 100 nM, and ssDNA (25 mer), of which the concentration had been adjusted to 1 µM, were held at 95°C for 10 min to denature the DNA. The denatured DNA was filled in an amount of 5 µl into each of the openings in the seal on the glass, and the assembly was held at 95°C for 10 min to evaporate the solvent. Thereafter, ultraviolet light was applied at 120 mJ with a UV cross linker (model CL-1000, manufactured by UVP corporation) to immobilize the biotin-labeled ssDNA and the ssDNA onto the glass substrate. The seal was then peeled off from each of the glass substrates. Each of the glass substrates was shaken in a 0.2% SDS solution three times each for 15 min and was rinsed with ultrapure water while replacing the ultrapure water with fresh ultrapure water three times. These glass substrates were immersed in boiling water for 2 min and were taken out. Air was blown against the glass substrates to blow away the residual water. Subsequently, the glass substrates were immersed in absolute ethanol at 4°C for one min for dehydration, and air was blown against the glass substrates to blow away the residual ethanol. Thus, a working electrode with biotin-labeled DNA and ssDNA immobilized thereonto was obtained.

### (2) Preparation of working electrode with Cy3-labeled streptavidin immobilized thereonto

A pressure-sensitive adhesive seal (thickness: 0.5 mm) having openings with a diameter of 3 mm was placed on and was brought into close contact with the working electrode, with biotin-labeled DNA and ssDNA immobilized thereonto, prepared in the above step (1) at the same placed as in the above step (1). Subsequently, a Cy3-labeled streptavidin solution (solvent: water) adjusted to 1 µg/ml was filled in an amount of 5 µl into each of the openings in the seal on the glass, and the assembly was held at 37°C for 3 min, was rinsed with water, and was dehydrated to obtain a streptavidin-immobilized working electrode.

### (3) Preparation of immersion electrolyte pad, condensate electrolyte pad, and electrolyte solution

An aqueous tetrapropylammonium iodide (NPr₄I) solution adjusted to 0.2 M was provided as an electrolyte solution. Further, a 0.9 mm-thick blotting filter paper (manufactured by ATTO CORPORATION) cut into a size of 42 mm × 16 mm was immersed in 500 µl of the electrolyte solution to prepare an immersion electrolyte pad. Further, in the same manner as in the immersion electrolyte pad, a 0.9 mm-thick blotting filter paper cut into a size of 42 mm × 16 mm was immersed in 500 µl of the electrolyte solution, and the filter paper was then dried at 95°C for 10 min to prepare a condensate electrolyte pad. In use of the condensate electrolyte pad, 300 µl of water was added to the condensate electrode pad immediately before photocurrent detection.

### (4) Measurement of photocurrent

### (i) Detection of photocurrent with electrolyte pad

A typical exploded view of a sensor unit using an electrolyte pad is shown in Fig. 30. In order to construct the sensor unit shown in Fig. 30, a streptavidin-immobilized working electrode 51 prepared in step (1) and step (2) and a counter electrode 82 of a glass plate with platinum vapor-deposited thereon were provided. The electrolyte pad 83 prepared in step (3) was held between and brought into close contact with both the electrodes. In this case, in use of the condensate electrolyte pad, as described above, 300 µl of water was added to the condensate electrolyte pad immediately before photocurrent detection. At that time, the working electrode and the counter electrode were disposed so that the DNA-immobilized surface of the working electrode faced the platinum-deposited surface of the counter electrode. In such a state that both the electrodes were connected to an electrochemical analyzer, light emitted from a laser beam source 84 (green laser beams at an output of 60 mW, a diameter in an irradiated area of 1 mm, and a wavelength of 532 nm) was applied to the working electrode, and the current value observed at that time was recorded. In Fig. 30, numeral 85 designates a pressing member, numeral 86 a counter member, numeral 87 an electrolyte, numeral 88 biotin-labeled DNA, and numeral 89 Cy3-labeled streptavidin.

### (ii) Detection of photocurrent using electrolyte solution (Reference Example)

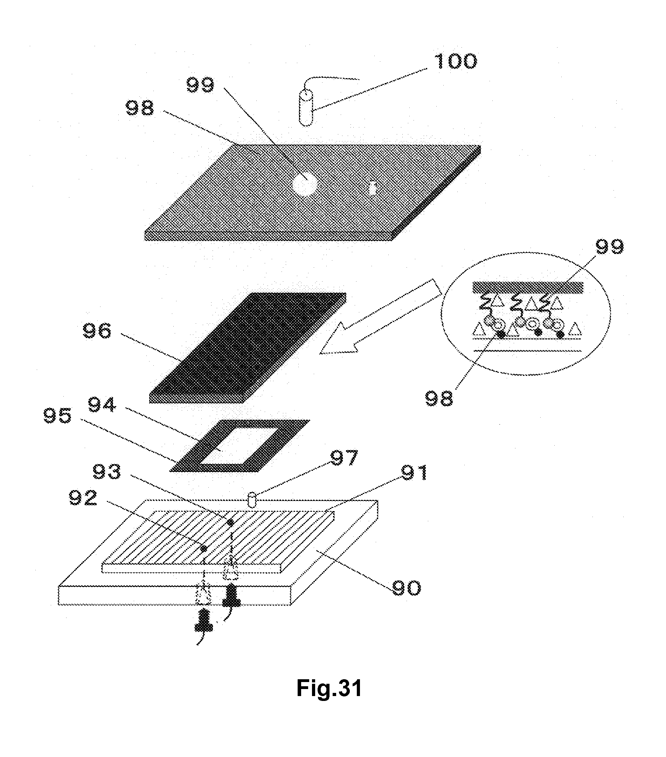

Fig. 31 is a typical exploded view of a flow cell-type measuring cell used when an electrolyte solution was used. In the flow cell-type measuring cell shown in Fig. 31, a counter electrode 91 is provided on a substrate 90. An electrolyte solution or cleaning solution supplying hole 92 and an electrolyte solution or cleaning solution discharge hole 93 are provided in the substrate 90. An insulating spacer 95 having an opening 94 for storing an electrolyte solution is disposed on the counter electrode 91. A working electrode 96 is provided on the insulating spacer 95. A contact 97 for a working electrode extends through the substrate 90 so as not to interfere with the counter electrode 91. Photocurrent is withdrawn through the contact 97 for a working electrode. A pressing member 98 is provided on the working electrode 96, and a through-hole 99 is provided in the pressing member 98. Light from the light source 100 is applied to the working electrode 96 through the through-hole 90. An ammeter is connected between the working electrode 96 and the counter electrode 91, and photocurrent that flows in the system upon light irradiation is measured with the ammeter.

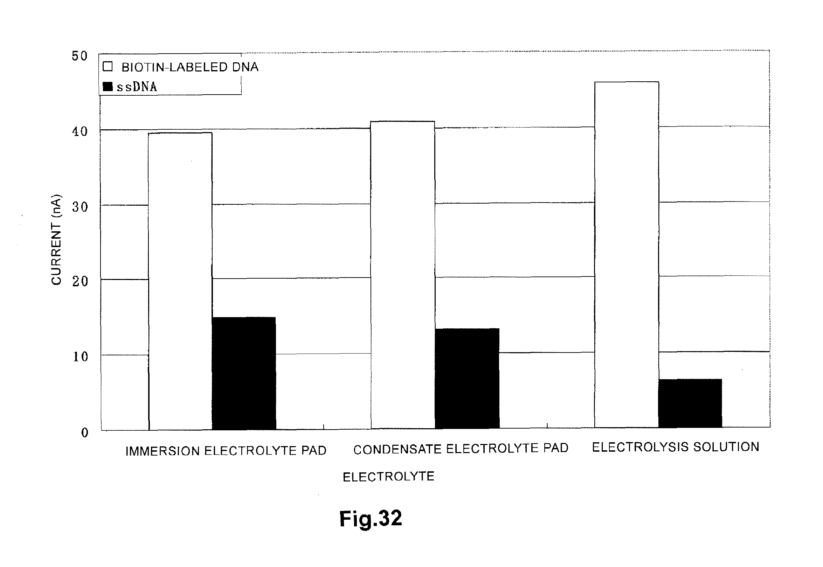

The results are shown in Fig. 32. As shown in Fig. 32, photocurrent derived from specific binding between Cy3-labeled streptavidin and biotin-labeled DNA was detected when any of the immersion electrolyte pad, the condensate electrolyte pad, and the electrolyte solution was used.

### Example 2: Specific detection of protein with reaction pad and spacer

### (1) Preparation of working electrode with Cy3-labeled streptavidin immobilized thereonto

In the same manner as in step (1) of Example 1, a working electrode with biotin-labeled DNA and ssDNA immobilized thereonto was prepared. Thereafter, the working electrode was brought into contact with an aqueous Cy3-labeled streptavidin solution using a reaction pad and a spacer. When the reaction pad was used, a 0.34 mm-thick membrane filter (MILLIPORE: 5.0 µm, SVPP) cut into a size of 42 mm × 16 mm was placed on the working electrode with biotin-labeled DNA and ssDNA immobilized thereonto and 150 µl of an aqueous Cy3-labeled streptavidin solution adjusted to 1 µg/ml was added. When the spacer was used, a 1 mm-thick silicone rubber having an opening with a size of 42 mm × 16 mm was placed on the working electrode and 300 µl of an aqueous Cy3-labeled streptavidin solution adjusted to 1 µg/ml was added. The assemblies were allowed to stand for 3 min, were rinsed with water, and were dehydrated. Thereafter, photocurrent was detected in the same manner as in (i) in step (4) of Example 1. In the detection of photocurrent, an immersion electrolyte pad prepared in the same manner as in step (3) of Example 1 was used as the electrode pad.

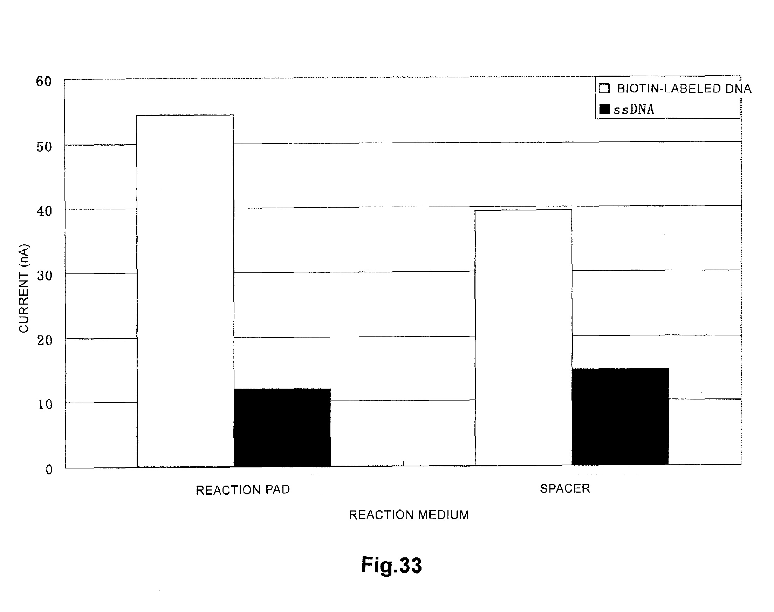

The results are shown in Fig. 33. As shown in Fig. 33, photocurrent derived from specific binding between Cy3-labeled streptavidin and biotin-labeled DNA was detected both when the reaction pad was used as the sample solution holding member and when the spacer was used as the sample solution holding member.

### Example 3: Specific detection of protein with dried reaction pad

### (1) Preparation of dried reaction pad

In the same manner as in step (1) of Example 2, two sheets of 0.34 mm-thick membrane filters (as described above) having a shape of 42 mm × 16 mm were provided, and 150 µl of an aqueous Cy3-labeled streptavidin solution adjusted to 1 µg/ml was added to each of the sheets. Thereafter, one of them was dried at 40°C for 2 hr, and the other sheet was lyophilized at -30°C for 15 min and was dried in vacuo for 2 hr.

### (2) Preparation of working electrode with Cy3-labeled streptavidin immobilized thereonto

In the same manner as in step (1) of Example 1, a working electrode with biotin-labeled DNA and ssDNA immobilized thereonto was prepared. Further, the heat-dried reaction pad and the lyophilized reaction pad were placed on the working electrode, with biotin-labeled DNA and ssDNA immobilized thereonto, prepared in step (1), and 150 µl of water was added. The assemblies were allowed to stand for 3 min, were rinsed with water, and were dehydrated followed by the preparation of a working electrode with Cy3-labeled streptavidin immobilized thereonto. For comparison, in the same manner as in step (1) of Example 2, 150 µl of an aqueous Cy3-labeled streptavidin solution adjusted to 1 µg/ml was added to a membrane filter on the working electrode, followed by standing for 3 min to prepare a working electrode with Cy3-labeled streptavidin immobilized thereonto. Thereafter, each electrode was used to detect photocurrent in the same manner as in step (4) of Example 1. In the detection of photocurrent, the immersion electrolyte pad prepared in the same manner as in step (3) of Example 1 was used as the electrolyte pad.

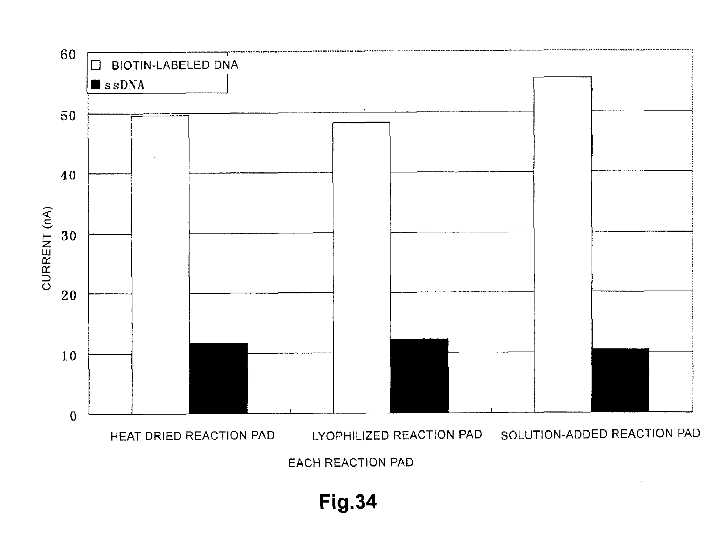

The results are shown in Fig. 34. As shown in Fig. 34, photocurrent derived from specific binding between Cy3-labeled streptavidin and biotin-labeled DNA was detected both when the heat-dried reaction pad was used and when the lyophilized reaction pad was used. As demonstrated in this working example, when a method is adopted in which a labeled protein is dried and held within a pad and a competition method or a sandwich method is used, an analyte can easily be quantitatively or qualitatively determined simply by adding a solution containing an analyte and the like and performing condensing.

### Example 4: Experiment on effect of electrolyte on specific reaction (Reference Example)

In the same manner as in step (1) of Example 1, a working electrode with biotin-labeled DNA and ssDNA immobilized thereonto was prepared and was then installed in a flow cell-type measuring cell shown in Fig. 31. A mixed solution (150 µl) prepared by mixing 75 µl of an aqueous tetrapropylammonium iodide (NPr₄I) solution adjusted to 0.4 M and 75 µl of an aqueous Cy3-labeled streptavidin solution adjusted to 2 µg/ml at a mixing ratio of 1 : 1 was supplied into the cell and was allowed to stand for 3 min. Thereafter, the mixed solution was discharged, 150 µl of an aqueous tetrapropylammonium iodide (NPr₄I) solution adjusted to 0.2 M was supplied into the cell, and photocurrent was detected. For comparison, 150 µl of an aqueous Cy3-labeled streptavidin solution adjusted to 1 µg/ml was supplied into the cell and was allowed to stand for 3 min. Thereafter, 150 µl of an aqueous tetrapropylammonium iodide (NPr₄I) solution adjusted to 0.2 M was supplied into the cell, and photocurrent was detected.

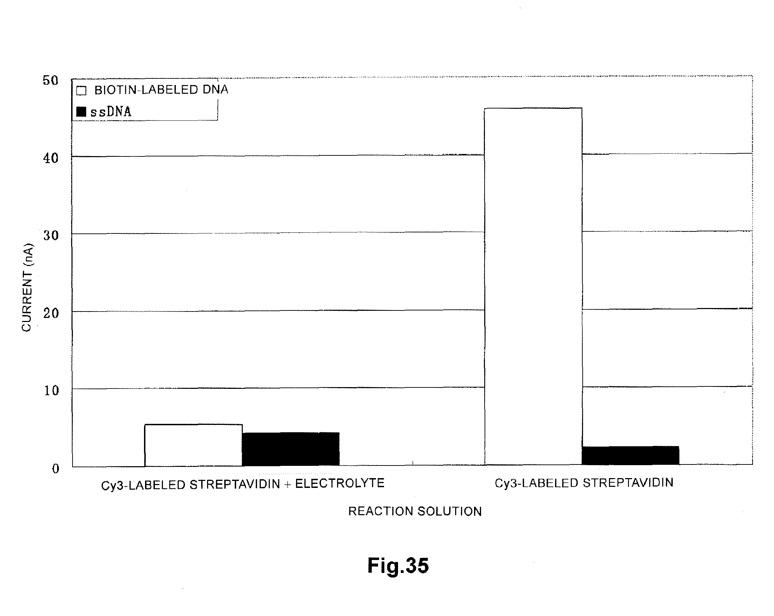

The results are shown in Fig. 35. As shown in Fig. 35, when an electrolyte was present in a reaction solution, photocurrent was dramatically decreased, and photocurrent was not specifically detected. This fact suggests that the electrolyte inhibits a specific reaction of protein.

### Example 5: Detection of protein with different reaction pads

In the same manner as in step (1) of Example 1, a working electrode with biotin-labeled DNA and ssDNA immobilized thereonto was prepared. Thereafter, different reaction pads were brought into contact with an aqueous Cy3-labeled streptavidin solution. The reaction pads used were a 0.34 mm-thick membrane filter (MILLIPORE: 5.0 µm, SVPP) cut into a size of 42 mm × 16 mm, a 0.21 mm-thick blotting filter paper (manufactured by WHATMAN Co,. Ltd.), and a 0.26 mm-thick glass fiber filter paper (GF/A, manufactured by WHATMAN Co,. Ltd.). Each of the reaction pads were placed on the working electrode with biotin-labeled DNA and ssDNA immobilized thereonto, and an aqueous Cy3-labeled streptavidin solution adjusted to 1 µg/ml was added. The amount of the solution added was 150 µl for the membrane filter, was 150 µl for the blotting filter paper, and was 700 µl for the glass fiber filter paper. The assemblies were allowed to stand for 3 min, were rinsed with water, and were dehydrated. Thereafter, in the same manner as in step (4) of Example 1, photocurrent was detected. In this case, the immersion electrolyte pad prepared in the same manner as in step (3) of Example 1 was used as the electrolyte pad.

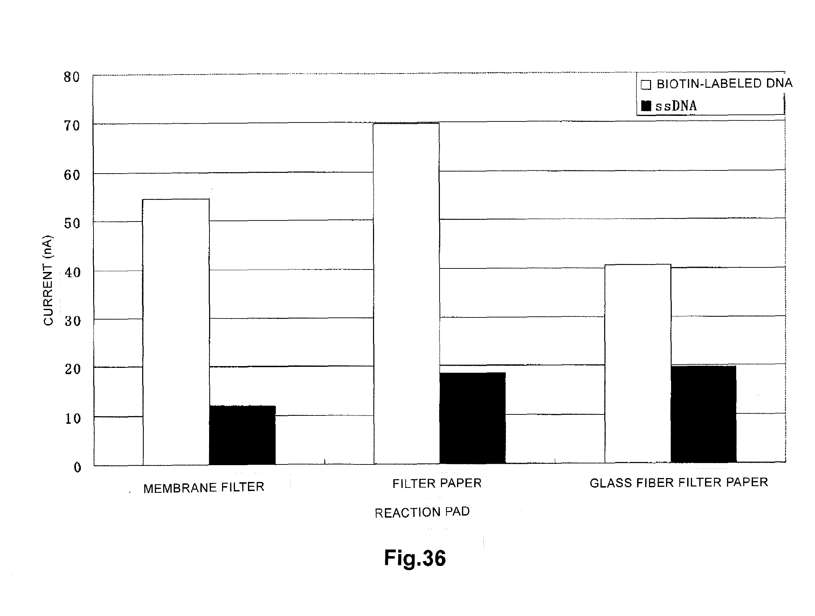

The results are shown in Fig. 36. As shown in Fig. 36, photocurrent derived from specific binding between Cy3-labeled streptavidin and biotin-labeled DNA was detected even when any of the above reaction pads was used.

### Example 6: Experiment on effect of salt, surfactant, and protein present as mixture in detection of photocurrent

Photocurrent was detected in the same manner as in Example 1, except that the electrolyte solution to be supplied was changed to 150 µl of a mixed solution prepared by mixing 75 µl of an aqueous tetrapropylammonium iodide (NPr₄I) solution adjusted to 0.4 M and 75 µl of an aqueous NaCl solution adjusted to 300 mM at a mixing ratio of 1 : 1, 150 µl of a mixed solution prepared by mixing 75 µl of an aqueous tetrapropylammonium iodide (NPr₄I) solution adjusted to 0.4 M and 75 µl of an aqueous Tween 20 solution adjusted to 0.1% at a mixing ratio of 1 : 1, and 150 µl of a mixed solution prepared by mixing 75 µl of an aqueous tetrapropylammonium iodide (NPr₄I) solution adjusted to 0.4 M and 75 µl of an aqueous streptavidin solution adjusted to 2 µg/ml at a mixing ratio of 1 : 1. For comparison, photocurrent was detected using an aqueous tetrapropylammonium iodide (NPr₄I) solution adjusted to 0.2 M as an electrolyte solution.

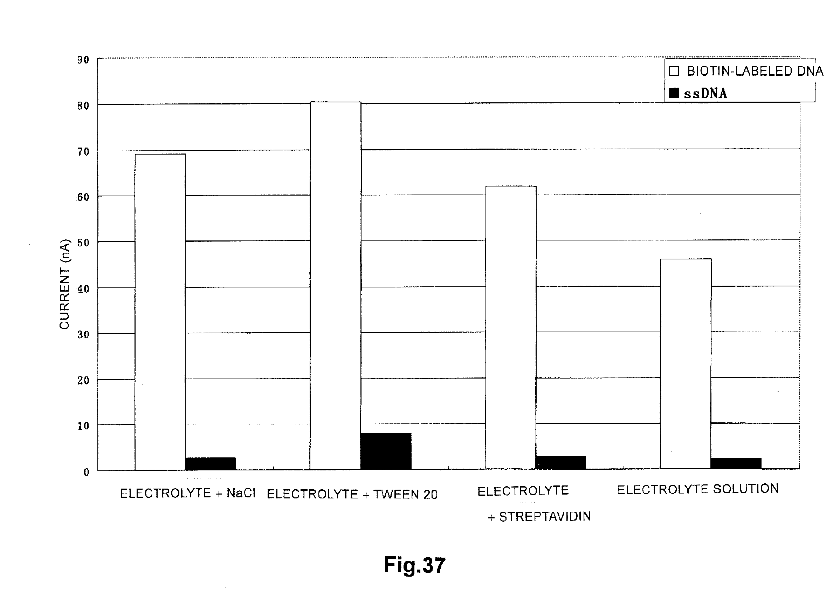

The results are shown in Fig. 37. As shown in Fig. 37, photocurrent derived from specific binding between Cy3-labeled streptavidin and biotin-labeled DNA was detected even when NaCl as salt, Tween 20 as a surfactant, or streptavidin as a protein was also present as a mixture. The results show that, according to the method and sensor unit according to the present invention, even when, in the detection of photocurrent in actual use, for example, salt contained in blood, protein other than the analyte, and a surfactant contained in a diluting solution are also contained in a sample, photocurrent can be satisfactorily detected.

### Example 7: Specific detection of protein in the presence of reaction pad and reaction solution

An example in which an immersion electrolyte pad or a condensate electrolyte pad was added in the presence of a reaction pad or a reaction solution will be described. At the outset, in the same manner as in Example 2, a working electrode with biotin-labeled DNA and ssDNA immobilized thereonto was prepared. Thereafter, the working electrode was brought into contact with an aqueous Cy3-labeled streptavidin solution using a reaction pad and a spacer. When the reaction pad was used, a 0.34 mm-thick membrane filter (MILLIPORE: 5.0 µm, SVPP) cut into a size of 42 mm × 16 mm was placed on the working electrode with biotin-labeled DNA and ssDNA immobilized thereonto and 150 µl of an aqueous Cy3-labeled streptavidin solution adjusted to 1 µg/ml was added. When the spacer was used, a 1 mm-thick silicone rubber having an opening with a size of 42 mm × 16 mm was placed on the working electrode and 300 µl of an aqueous Cy3-labeled streptavidin solution adjusted to 1 µg/ml was added. They were allowed to stand for 3 min. Thereafter, an immersion electrolyte pad, a condensate electrolyte pad, or an electrolyte solution that had been prepared in the same manner as in step (3) of Example 1 except for the use of a 0.34 mm-thick membrane filter (MILLIPORE: 5.0 µm SVPP) cut into a size of 42 mm × 16 mm was added, and photocurrent was detected in the same manner as in step (4) of Example 1. When the condensate electrolyte pad was used, water was not added and, in this case, the condensing was carried out with the aqueous Cy3-labeled streptavidin solution as the reaction solution.

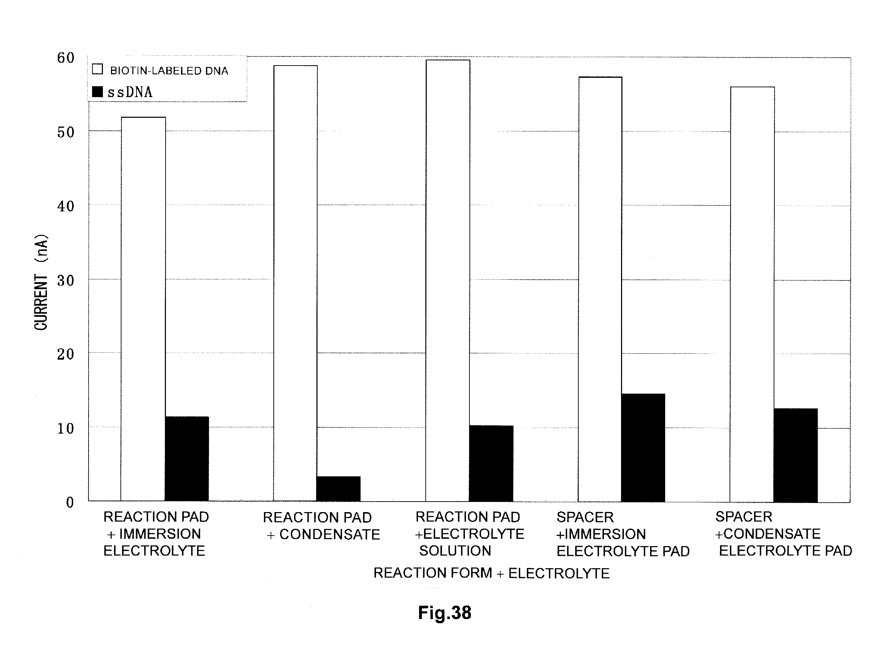

The results are shown in Fig. 38. As shown in Fig. 38, photocurrent derived from specific binding between Cy3-labeled streptavidin and biotin-labeled DNA was detected in all the combinations. The results demonstrate that, according to the method and sensor unit according to the present invention, protein can be specifically detected in the presence of a reaction pad and a reaction solution and, in the detection of photocurrent, condensing of the condensate electrolyte pad by a reaction solution or a reaction pad is possible. In this working example, the "reaction pad + immersion electrolyte pad" and the "reaction pad + condensate electrolyte pad" correspond to the sensor unit in the first embodiment, the "reaction pad + electrolyte solution" corresponds to the sensor unit in the fourth embodiment, and the "spacer + immersion electrolyte pad" and the "spacer + condensate electrolyte pad" correspond to the sensor unit in the third embodiment.

### Example 8: Studies on various conditions of electrolyte pad

### (1) Preparation of working electrode with dye-labeled DNA immobilized thereonto

A fluorine-doped tin oxide (F-SnO₂: FTO) coated glass (manufactured by AI Special Glass Company, U film, sheet resistance: 12 Ω/□, and shape: 50 mm × 26 mm) was provided as a glass substrate for a working electrode. This glass substrate was ultrasonically cleaned in acetone for 15 min and subsequently in ultrapure water for 15 min to remove contaminants and residual organic matter. The glass substrate was shaken in a 5 M aqueous sodium hydroxide for 15 min. Thereafter, shaking of the glass substrate in ultrapure water for 5 min was repeated three times while replacing water with fresh water for each shaking to remove sodium hydroxide. The glass substrate was taken out, and air was blown against the glass substrate to blow away the residual water. The glass substrate was then immersed in anhydrous methanol for dehydration.

3-Aminopropyltrimethoxysilane (APTMS) was added to a solvent composed of 95% methanol and 5% ultrapure water to bring the APTMS concentration to 2% by volume, and the mixture was stirred at room temperature for 5 min to prepare a solution for coupling treatment. The above glass substrate was immersed in the solution for coupling treatment, and was then slowly shaken for 15 min. The glass substrate was then taken out and was shaken approximately 10 times in methanol to remove excess solution for coupling treatment. This procedure was repeated three times while replacing methanol with fresh methanol for each time. Thereafter, the glass substrate was held at 110°C for 30 min to bind the coupling agent to the glass substrate. The glass substrate was cooled at room temperature, and a pressure-sensitive adhesive seal (thickness: 0.5 mm) having openings with a diameter of 3 mm was placed on and brought into close contact with the glass substrate. Subsequently, 5'-terminal rhodamine-labeled ssDNA (25 mer), of which the concentration had been adjusted to 100 nM, and non-labeled ssDNA (24 mer), of which the concentration had been adjusted to 100 nM, were held at 95°C for 10 min. Immediately after that, these ssDNAs were transferred onto ice and were held in this state for 10 min to denature the DNAs. The denatured DNAs were filled in an amount of 5 µl into each of the openings in the seal on the glass, and the assembly was held at 95°C for 10 min to evaporate the solvent. Thereafter, ultraviolet light was applied at 120 mJ with a UV cross linker (model CL-1000, manufactured by UVP corporation) to immobilize the labeled ssDNA and non-labeled ssDNA onto each of the glass substrates. The seal was then peeled off from each of the glass substrates. Each of the glass substrates was shaken in a 0.2% SDS solution three times each for 15 min and was rinsed with ultrapure water while replacing the ultrapure water with fresh ultrapure water three times. These glass substrates were immersed in boiling water for 2 min and were taken out. Air was blown against the glass substrates to blow away the residual water. Subsequently, the glass substrates were immersed in absolute ethanol at 4°C for one min for dehydration, and air was blown against the glass substrates to blow away the residual ethanol. Thus, a working electrode with dye-labeled DNA immobilized thereonto and a working electrode with ssDNA immobilized thereonto were obtained. The base sequences of the probe DNAs were as follows. 5' Terminal rhodamine-labeled ssDNA (probe 1):
5'-Rho-GCGGCATGAACCTGAGGCCCATCCT-3' (Sequence No. 1)
Non-labeled ssDNA (probe 2):
5'-TTGAGCAAGTTCAGCCTGGTTAAG-3' (Sequence No. 2)

### (2) Preparation of electrolyte-containing pad

Aqueous solutions containing tetrapropylammonium iodide (NPr4I) (0.2 M, 0.4 M, and 0.6 M) were prepared. A 0.9 mm-thick blotting filter paper (CB-13A; manufactured by ATTO CORPORATION) cut into a size of 26 mm × 20 mm was immersed in the aqueous solution and was lightly hydro-extracted to prepare an electrolyte-containing pad.

### (3) Studies on electrolyte concentration

The dye-labeled DNA-immobilized working electrode prepared in the above step (1), and a counter electrode formed of a glass plate with platinum vapor-deposited thereon were provided. The electrolyte-containing pad prepared in the above step (2) was held between and brought into close contact with both the electrodes. In this case, the working electrode and the counter electrode were disposed so that the ssDNA-immobilized surface of the working electrode faced the platinum-deposited surface of the counter electrode. In such a state that both the electrodes were connected to an electrochemical analyzer, the working electrode was irradiated with light from a laser source (green laser having output of 60 mW, irradiation region diameter of 1 mm, and wavelength of 530 nm) and the current value observed at this time was recorded.

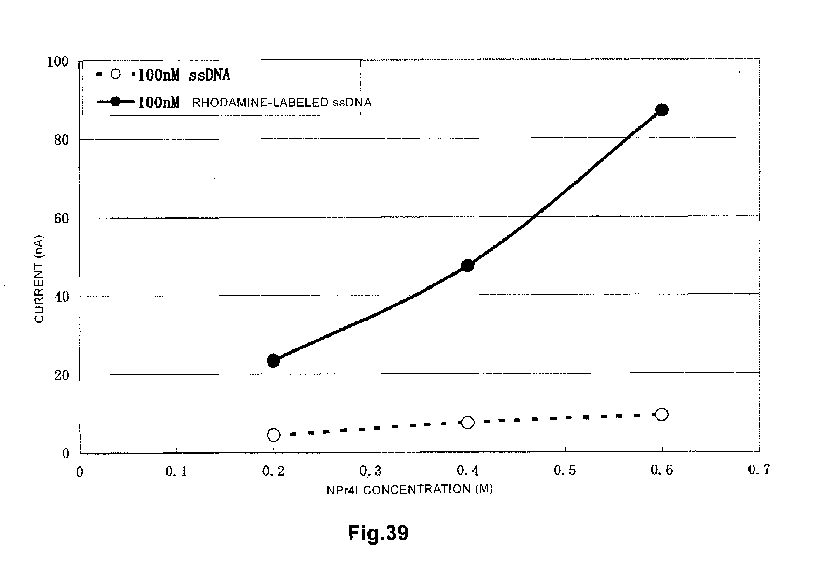

The results are shown in Fig. 39. As can be seen from Fig. 39, the photocurrent was increased dependent upon the concentration of tetrapropylammonium iodide. However, it was demonstrated that tetrapropylammonium iodide even in any concentration can be used in the measurement.

### (4) Studies on different electrolytes

Photocurrent was measured using different electrolytes. Specifically, a filter paper was used as a water-absorptive substrate, and the concentration of each of reducing agents was fixed to 0.2 M to prepare electrolyte-containing pads. NaI, KI, CaI₂, LiI, NH₄I, tetrapropylammonium iodide (NPr₄I), sodium thiosulfate (Na₂S₂O₃), and sodium sulfite (Na₂SO₃) were used as electrolytes. Electrolyte solutions containing these different electrolytes and water were prepared. A 0.9 mm-thick blotting filter paper (CB-13A; manufactured by ATTO CORPORATION) cut into a size of 26 mm × 20 mm was immersed in the electrolyte solutions, followed by light hydro-extraction to prepare electrolyte-containing pads. Dye-labeled DNA-immobilized working electrodes were prepared in the same manner as in step (1), except that a solution having a rhodamine-labeled ssDNA concentration of 10 nM was used. Working electrodes prepared using a solution having a rhodamine-non-labeled ssDNA concentration of 100 nM were also used. Photocurrent was measured in the same manner as in the above step (3).

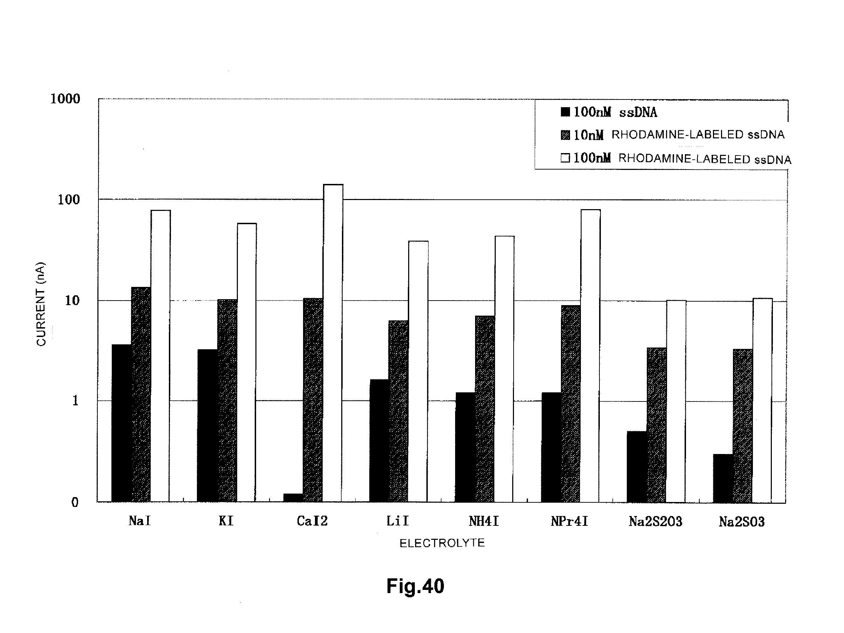

The results are shown in Fig. 40. As can be seen from Fig. 40, for all the examined electrolytes, photocurrent was increased dependent upon the amount of ssDNA immobilized, indicating that these electrolytes are usable in the measurement.

### (5) Studies on thickness of electrolyte-containing pad

Dye-labeled DNA-immobilized working electrodes were prepared in the same manner as in the above step (1), except that two types of concentrations of 100 nM and 1 µM were used as the concentration of ssDNA to be immobilized. An electrolyte solution was prepared from 0.2 M tetrapropylammonium iodide (NPr₄I) and water. A 0.9 mm-thick blotting filter paper (CB-13A; manufactured by ATTO CORPORATION) was then provided. This filter paper was used in three different thicknesses, i.e., as a single sheet, a two-sheet stack prepared by placing two sheets of the filter paper on top of each other, and a three-sheet stack prepared by placing three sheets of the filter paper on top of each other, each of which was immersed in the electrolyte solution to prepare electrolyte-containing pads. Thereafter, photocurrent was measured in the same manner as in the above step (3), except that the working electrodes different from each other in immobilization concentration and the electrolyte-containing pads different from each other in thickness were used.

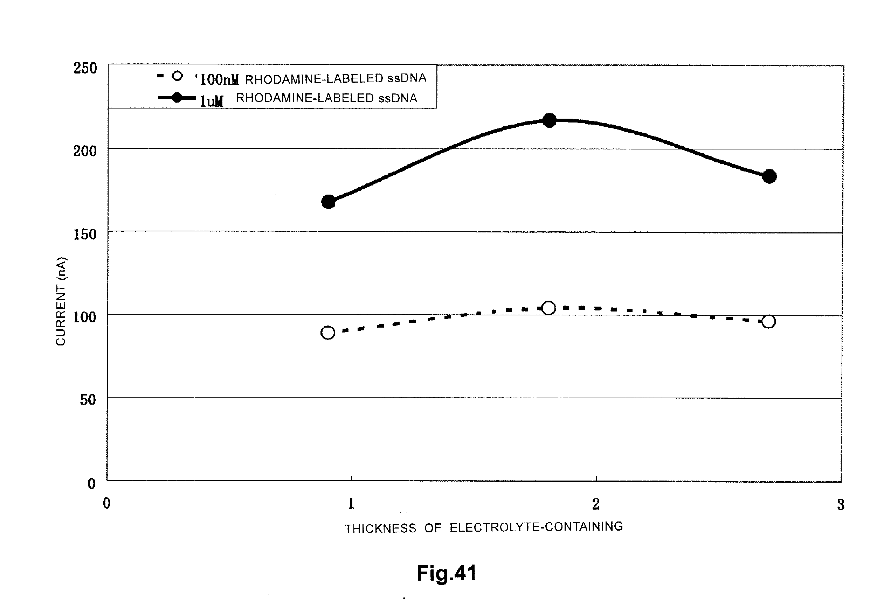

The results are shown in Fig. 41. As can be shown from Fig. 41, the influence of the thickness of the electrolyte-containing pad on photocurrent values was hardly observed over the whole thickness range (0.9 mm to 2.7 mm), indicating that increasing the thickness of the electrolyte-containing pad from the viewpoint of ensuring satisfactory strength has no adverse effect on the detection of photocurrent values.

### (6) Studies on different water-absorptive materials

Photocurrent was measured in the same manner as in the above step (3), except that a 0.9 mm-thick blotting filter paper (CB-13A; manufactured by ATTO CORPORATION), a felt (thickness: 1 mm, density: 0.00049 g/mm³), a cardboard (thickness: 0.5 mm, density: 0.00071 g/mm³), a glass fiber filter paper (GF/D; Whatman; thickness: 0.68 mm), a coated sheet including pulp fibers and synthetic fibers (thickness: 0.14 mm, density: 0.00111 g/mm³), and a membrane filter composed mainly of fluororesin (JCWP09025; MILLIPORE: thickness: 0.1 mm) were used as water-absorptive materials. Dye-labeled DNA-immobilized working electrodes were prepared in the same manner as in the above step (1), except that solutions having a rhodamine-labeled ssDNA concentration of 100 nM and a rhodamine-labeled ssDNA concentration of 1 µM were used. Working electrodes prepared using a solution having a non-labeled ssDNA concentration of 100 nM were also used. A 2 M aqueous tetrapropylammonium iodide (NPr₄I) solution was used as the electrolyte solution.

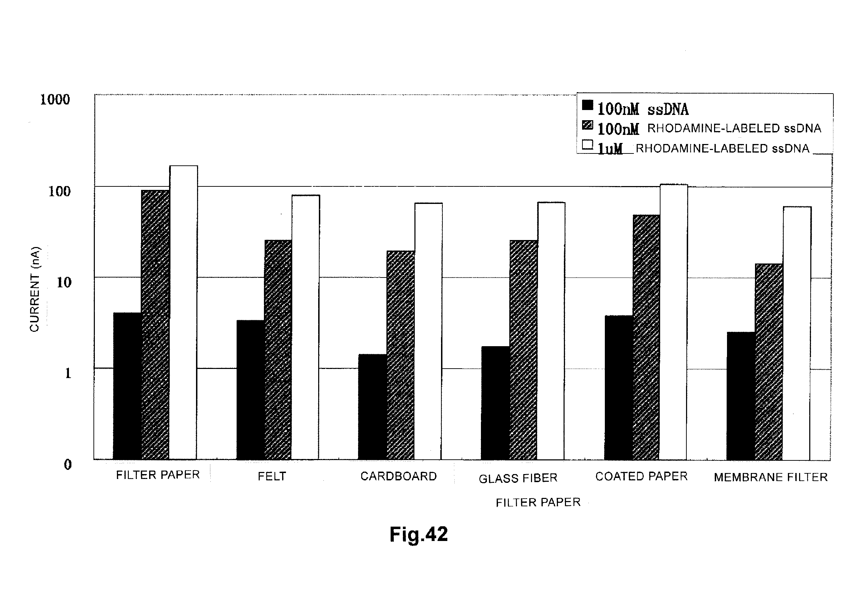

The results are shown in Fig. 42. As can be seen from Fig. 42, for all the water-absorptive materials, the photocurrent was increased dependent upon the amount of ssDNA immobilized, indicating that these water-absorptive materials are usable as the electrolyte-containing pad.

### (7) Studies on water content of electrolyte-containing pad

### (i) Measurement of water content

At the outset, a 0.4 M aqueous tetrapropylammonium iodide (NPr₄I) solution (500 µl) was dropped on a 0.9 mm-thick blotting filter paper (CB-13A; manufactured by ATTO CORPORATION) cut into a size of 26 mm × 20 mm to fully impregnate the filter paper with the electrolyte solution. Thus, filter papers impregnated with the electrolyte solution were prepared. Next, the impregnated filter papers were dried at 50°C for 0 hr, 0.25 hr, 0.5 hr, 1 hr, 1.25 hr, and 1.5 hr, respectively. The weight of the filter papers after the drying was measured. The weight of the tetrapropylammonium iodide was removed from the measured weight of the filter papers, and the water content of the filter paper per mm³ was then calculated. The ratio of (water content per mm³)/(density of filter paper) was calculated to obtain the water content of the electrolyte-containing pad. It should be noted that the density of the blotting filter paper is 0.00049 g/mm³.

### (ii) Measurement of photocurrent

Photocurrent was detected in the same manner as in Example 1, except that the electrolyte-containing pads having the respective water contents prepared in the above step (i) were used.

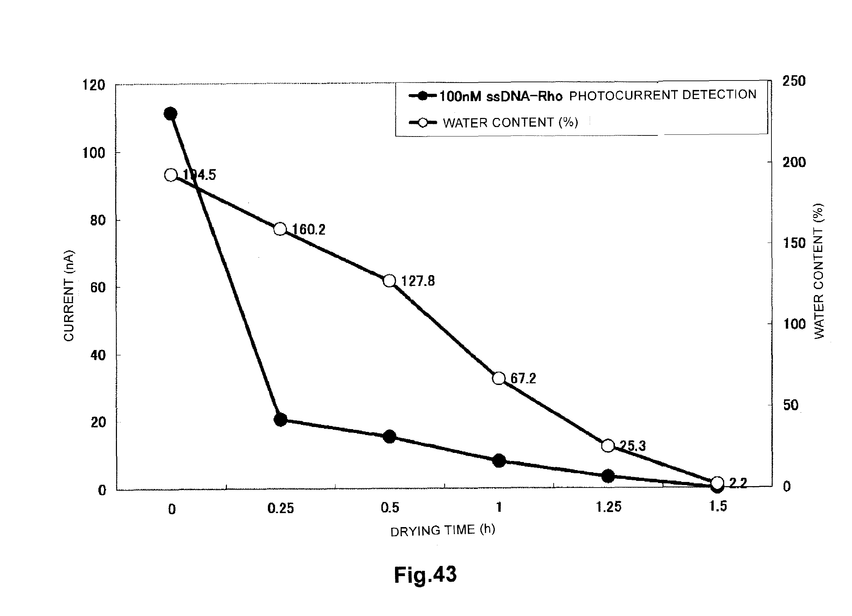

The results are shown in Fig. 43. As can be seen from Fig. 43, the photocurrent increases with increasing the water content of the electrolyte-containing pad. When the water content was 2.2%, photocurrent could not be detected, whereas, when the water content was 25.3%, photocurrent could be detected. These results show that, preferably, the electrolyte-containing pad which can detect photocurrent has a water content of at least 20%.

### Example 9: Immunoassay using reaction pad and condensate electrolyte pad

### (1) Preparation of electrode

A fluorine-doped tin oxide (F-SnO₂: FTO) coated glass (manufactured by AI Special Glass Company, U film, sheet resistance: 12 Ω/cm², and shape: 50 mm × 26 mm) was provided as a substrate for a working electrode. An electrode comprising the FTO electrode and a 50 nm-thick ZnO film sputtered on the FTO electrode (200 W, sputtering time 8 min, sputter rate 6.25 nm/min) was provided (film thickness: estimated roughly from sputter rate). This electrode was ultrasonically cleaned with acetone, ultrapure water, and acetone successively in that order each for one min, was immersed in a 1 M nitric acid solution (pH 0.2), and was shaken for 5 min. Thereafter, the electrode was thoroughly rinsed with ultrapure water to prepare a working electrode.

### (2) Immobilization of primary antibody

A pressure-sensitive adhesive seal (thickness: 0.5 mm) having openings with a diameter of 3 mm was placed on and brought into close contact with the electrode thus prepared. A goat-derived antibody solution adjusted to 10 µg/ml (10 mM phosphate buffer [pH7] 0.05% Tween 20, 250 mM NaCl) was dropped in an amount of 5 µl into each of the openings, followed by incubation at 37°C for 10 min. Thereafter, the electrode was shaken and washed in ultrapure water for 10 min.

### (3) Immunoassay

In the primary antibody-immobilized electrode prepared in the above step (2), the applied seal was peeled off, and a reaction pad (PVDF membrane (MILLIPORE: 5.0 µm, SVPP) was installed thereon. Cy5 labeled anti-goat antibody adjusted to 1 ng/ml, 10 ng/ml, 100 ng/ml, and 1 µg/ml (10 mM phosphate buffer, 0.05% Tween 20, 150 mm NaCl) was dropped each in an amount of 100 µl on the reaction pad, followed by incubation at 37°C for 10 min.

### (4) Measurement of photocurrent

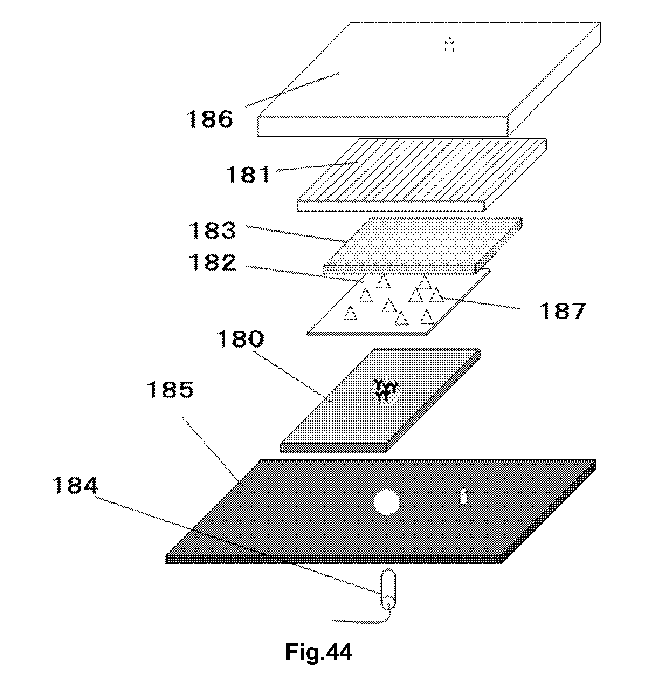

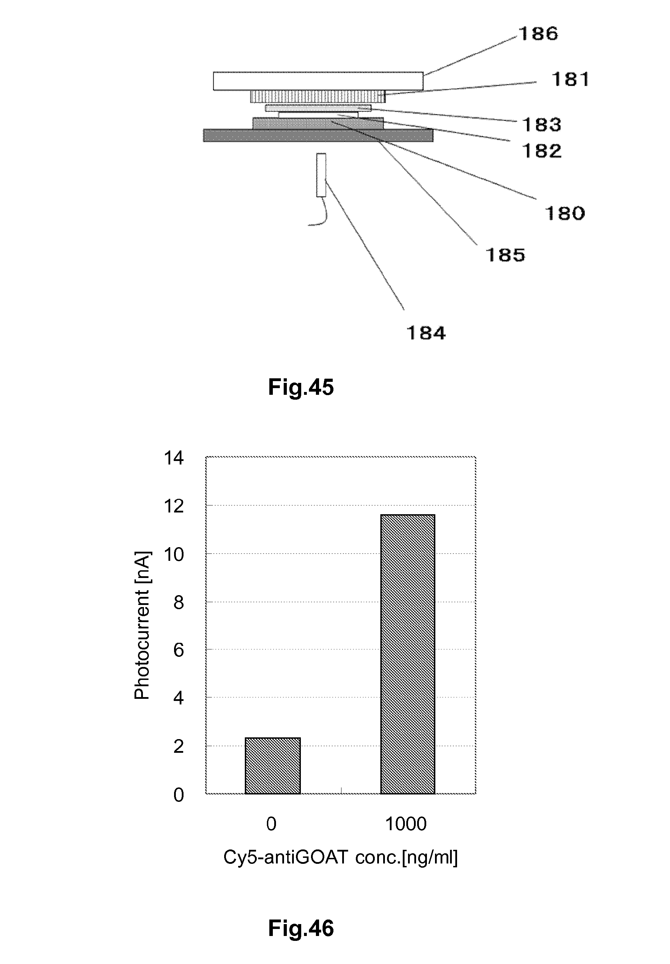

Fig. 44 is a typical exploded view of a sensor unit in one embodiment of the present invention used in this Example, and Fig. 45 is a cross-sectional view of the sensor unit shown in Fig. 44 when the photocurrent is detected. An analyte-bound working electrode 180, with the reaction pad remaining held thereon, prepared in the above steps (1) and (2) and a counter electrode 181 formed of a glass sheet with platinum vapor-deposited thereon were provided. A condensate electrolyte pad 183 prepared as described in Example 1 was superimposed in the dried state on the reaction pad to perform condensing the electrolyte pad using water held in the reaction pad 182. Thereafter, the analyte-bound working electrode with the reaction pad and the condensate electrolyte pad held thereon and the counter electrode were disposed so that the protein-immobilized surface of the working electrode faced the platinum-deposited surface of the counter electrode (see Figs. 44 and 45). In such a state that both the electrodes were connected to the electrochemical analyzer, the working electrode was irradiated with light from a laser beam source (red laser beams at an output of 120 mW, a diameter in an irradiated area of 1 mm, and a wavelength of 532 nm) 184, and the current value observed at that time was recorded. In Figs. 44 and 45, numeral 185 designates a pressing member, and numeral 186 a counter member.

The results are shown in Fig. 46. As can be seen from Fig. 46, photocurrent derived from a specific reaction between the antibody immobilized on the electrode and the analyte could be detected even when a method was used in which an analyte was reacted using a pad and the condensate electrolyte pad was condensed using the reaction solution.

### Example 10: Sandwich immunoassay using spacer and condensate electrolyte pad

### (1) Preparation of electrode

A fluorine-doped tin oxide (F-SnO₂: FTO) coated glass (manufactured by AI Special Glass Company, U film, sheet resistance: 12 Ω/cm², and shape: 50 mm × 26 mm) was provided as a substrate for a working electrode. An electrode comprising the FTO electrode and a 50 nm-thick ZnO film sputtered on the FTO electrode (200 W, sputtering time 8 min, sputter rate 6.25 nm/min) was provided (film thickness: estimated roughly from sputter rate). This electrode was ultrasonically cleaned with acetone, ultrapure water, and acetone successively in that order each for one min, was immersed in a 1 M nitric acid solution (pH 0.2), and was shaken for 5 min. Thereafter, the electrode was thoroughly rinsed with ultrapure water to prepare a working electrode.

### (2) Immobilization of primary antibody

A pressure-sensitive adhesive seal (thickness: 0.5 mm) having openings with a diameter of 3 mm was placed on and brought into close contact with the electrode thus prepared. An anti-AFP antibody (NB011, manufactured by Nippon Biotest Laboratories inc.) solution adjusted to 10 µg/ml (10 mM phosphate buffer [pH7] 0.05% Tween 20, 250 mM NaCl) was dropped in an amount of 5 µl into each of the openings, followed by incubation at 37°C for 10 min. Thereafter, the electrode was shaken and washed in ultrapure water for 5 min.

### (3) Immunoassay

In the primary antibody-immobilized electrode prepared in the above step (2), a mixed solution composed of AFP (antigen) and Cy5-labeled anti-AFP antibody was dropped in an amount of 5µl into each of the openings in the seal, followed by incubation at 37°C for 10 min. In this case, the Cy5-labeled anti-AFP antibody was adjusted to 50 µg/ml and AFP was adjusted to 1 µg/ml and 10 µg/ml before they were added.

### (4) Measurement of photocurrent

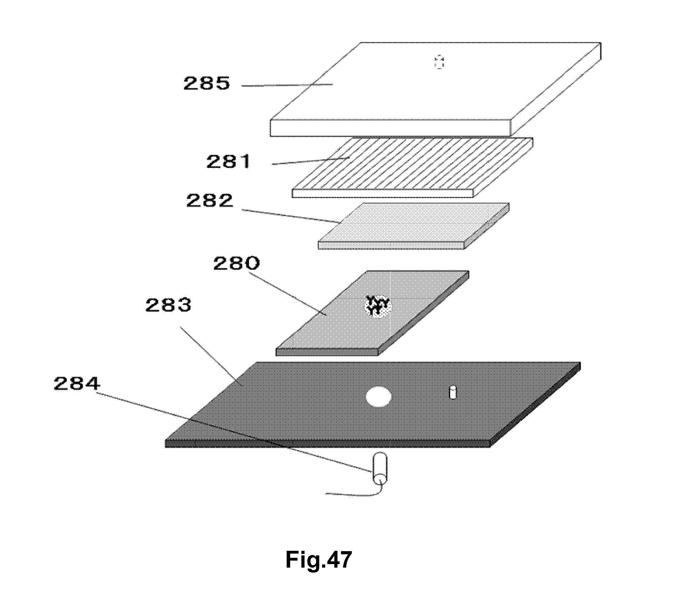

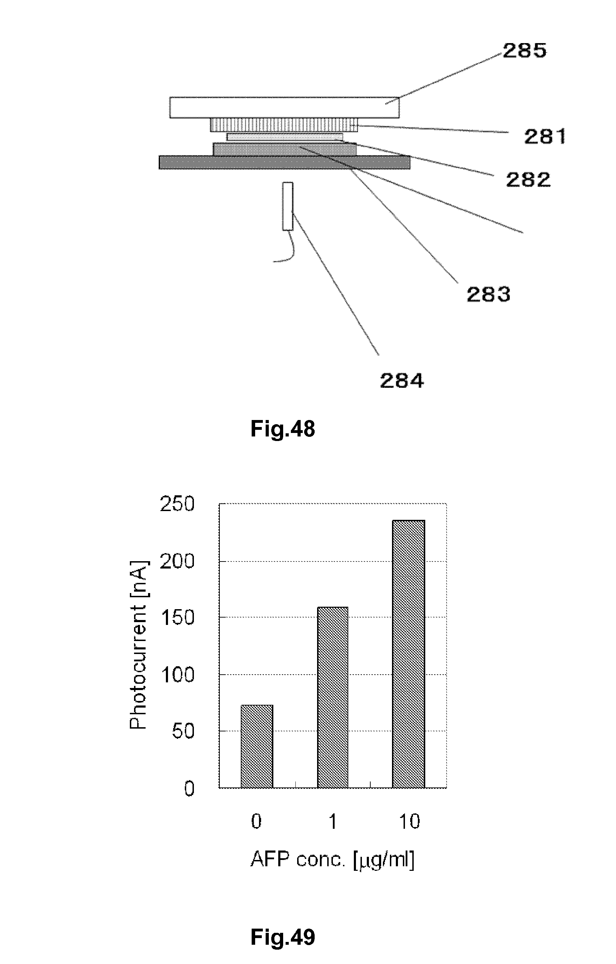

Fig. 47 is a typical exploded view of a sensor unit in one embodiment of the present invention used in this Example, and Fig. 48 is a cross-sectional view of the sensor unit shown in Fig. 47 when the photocurrent is detected. An analyte-bound working electrode 280, with the reaction solution remaining held thereon, prepared in the above steps (1) and (2) and a counter electrode 281 formed of a glass sheet with platinum vapor-deposited thereon were provided. A condensate electrolyte pad 282 prepared as described in Example 1 was superimposed in the dried state on the working electrode with the reaction solution held thereon by a seal, and the electrolyte pad was condensed using water in the reaction solution. Thereafter, the analyte-bound working electrode with the condensate electrolyte pad held thereon and the counter electrode were disposed so that the protein-immobilized surface of the working electrode faced the platinum-deposited surface of the counter electrode (see Figs. 47 and 48). In such a state that both the electrodes were connected to the electrochemical analyzer, the working electrode was irradiated with light from a laser beam source (red laser beams at an output of 120 mW, a diameter in an irradiated area of 1 mm, and a wavelength of 650 nm) 284, and the current value observed at that time was recorded. In Figs. 47 and 48, numeral 285 designates a pressing member, and numeral 283 a counter member.

The results are shown in Fig. 49. As can be seen from Fig. 49, photocurrent derived from a composite specific reaction between the antibody immobilized on the electrode and the analyte and the labeled antibody could be detected even when a method was adopted in which, after a reaction in the solution, the condensate electrolyte pad was condensed with the reaction solution.

## Claims

1. A method for specifically detecting an analyte using photocurrent that flows, across a working electrode and a counter electrode, attributable to ransfer of electrons from a photoexcited sensitizing dye to the working electrode, the working electrode having on its surface a probe substance capable of specifically binding directly or indirectly to the analyte, the method comprising:
bringing an analyte-containing sample solution and the sensitizing dye into contact with the surface of the working electrode to immobilize the sensitizing dye to the working electrode through specific binding;
supplying an electrolyte in situ while allowing the sample solution to be held without being removed, thereby bringing the working electrode and the counter electrode into contact with the electrolyte; and
irradiating the working electrode with light to photoexcite the sensitizing dye and thus to detect photocurrent that flows across the working electrode and the counter electrode.

2. The method according to claim 1, wherein the contact of the surface of the working electrode with the sample solution is carried out through a spacer having an opening for holding the sample solution on the surface of the working electrode.

3. The method according to claim 1, wherein the contact of the surface of the working electrode with the sample solution is carried out through a water-absorptive reaction pad for holding the reaction solution on the surface of the working electrode.

4. The method according to claim 3, wherein the reaction pad has a thickness of 0.01 to 10 mm.

5. The method according to claim 3 or 4, wherein the reaction pad is formed of at least one type of fibers selected from the group consisting of natural fibers, pulp fibers, regenerated fibers, glass fibers, and synthetic fibers.

6. The method according to any one of claims 3 to 5, wherein the reaction pad is formed of at least one filter selected from the group consisting of filter papers, membrane filters, glass filters, and filter cloths.

7. The method according to any one of claims 3 to 6, wherein the reaction pad has a water content of not less than 20% when the photocurrent is detected.

8. The method according to any one of claims 3 to 7, wherein the reaction pad further comprises the sensitizing dye.

9. The method according to any one of claims 1 to 8, wherein the sensitizing dye is pre-labeled on the analyte or a substance that can specifically bind to the probe substance.

10. The method according to any one of claims 1 to 9, wherein the electrolyte is supplied by adding an electrolyte solution.

11. The method according to any one of claims 1 to 9, wherein the electrolyte is supplied by previously impregnating the water-absorptive electrolyte pad with the electrolyte and bringing the electrolyte-impregnated water-absorptive electrolyte pad into contact with the sample solution to diffuse the electrolyte into the sample solution.

12. The method according to claim 11, wherein the electrolyte pad comprises at least one type of fibers selected from the group consisting of natural fibers, pulp fibers, regenerated fibers, glass fibers, and synthetic fibers.

13. The method according to claim 11 or 12, wherein the electrolyte pad is at least one filter selected from the group consisting of filter papers, membrane filters, glass filters, and filter cloths.

14. The method according to any one of claims 11 to 13, wherein the electrolyte pad has a water content of not less than 20% when the photocurrent is detected.

15. The method according to any one of claims 11 to 13, wherein a barrier film for blocking the diffusion of the electrolyte from the electrolyte pad into the reaction pad is previously provided between the reaction pad and the electrolyte pad and the barrier film is removed when the electrolyte is supplied.

16. The method according to any one of claims 1 to 15, wherein the working electrode and the counter electrode are disposed opposite to each other at least when light is applied.

17. The method according to any one of claims 1 to 15, wherein the working electrode and the counter electrode are disposed on the same plane.

18. A sensor unit for use in a method for specifically detecting an analyte using photocurrent that flows, across a working electrode and a counter electrode, attributable to transfer of electrons from a photoexcited sensitizing dye to a working electrode, the sensor unit comprising:
a working electrode that has on its surface a probe substance capable of specifically binding directly or indirectly to the analyte and can allow the sensitizing dye to be immobilized thereonto through specific binding by the contact of the working electrode with an analyte-containing sample solution and the sensitizing dye;
a counter electrode; and
a sample solution holding member that does not have a mechanism for removing the sample solution and holds the sample solution on the surface of the working electrode and the counter electrode.

19. The sensor unit according to claim 18, wherein the sample solution holding member is a spacer having an opening that can be filled with the sample solution.

20. The sensor unit according to claim 18, wherein the sample solution holding member is a water-absorptive reaction pad that can be impregnated with the sample solution.

21. The sensor unit according to claim 20, wherein the reaction pad has a thickness of 0.01 to 10 mm.

22. The sensor unit according to claim 20 or 21, wherein the reaction pad is formed of at least one type of fibers selected from the group consisting of natural fibers, pulp fibers, regenerated fibers, glass fibers, and synthetic fibers.

23. The sensor unit according to any one of claims 20 to 22, wherein the reaction pad is formed of at least one filter selected from the group consisting of filter papers, membrane filters, glass filters, and filter cloths.

24. The sensor unit according to any one of claims 20 to 23, wherein the reaction pad can have a water content of not less than 20%.

25. The sensor unit according to any one of claims 20 to 24, wherein the reaction pad further comprises the sensitizing dye.

26. The sensor unit according to any one of claims 18 to 25, wherein the water-absorptive electrolyte pad previously impregnated with the electrolyte is provided on the sample solution holding member in its area where the sample solution is held and which is located on its side remote from the working electrode so that the electrolyte pad is capable of contacting the area where the sample solution is to be held after the formation of the specific binding.

27. The sensor unit according to claim 26, wherein the electrolyte pad is formed of at least one type of fibers selected from the group consisting of natural fibers, pulp fibers, regenerated fibers, glass fibers, and synthetic fibers.

28. The sensor unit according to claim 26 or 27, wherein the electrolyte pad is formed of at least one filter selected from the group consisting of filter papers, membrane filters, glass filters, and filter cloths.

29. The sensor unit according to any one of claims 26 to 28, wherein the electrolyte pad can have a water content of not less than 20%.

30. The sensor unit according to any one of claims 26 to 29, wherein a barrier film for blocking the diffusion of the electrolyte from the electrolyte pad into the reaction pad is removably provided between the reaction pad and the electrolyte pad.

31. The sensor unit according to any one of claims 18 to 30, wherein the working electrode and the counter electrode are disposed opposite to each other, and the sample solution holding member and the electrolyte pad, if any, are held between the working electrode and the counter electrode.

32. The sensor unit according to any one of claims 18 to 30, wherein the working electrode and the counter electrode are disposed on the same plane, and the sample solution holding member is disposed in contact with the plane.

33. The sensor unit according to any one of claims 18 to 32, wherein a holding member and a counter member are provided opposite to each other to hold therebetween a laminate of the working electrode, the counter electrode, the sample solution holding member, and the electrolyte pad, if any.

34. The sensor unit according to any one of claims 16 to 29, for use in the method according to any one of claims 1 to 17.

35. A measuring device for use in a method for specifically detecting an analyte using photocurrent that flows, across a working electrode and a counter electrode, attributable to transfer of electrons from a photoexcited sensitizing dye to a working electrode, the measuring device comprising:
a sensor unit according to any one of claims 18 to 34;
a light source that applies light to the working electrode; and
an ammeter that detects photocurrent that flows, across the working electrode and the counter electrode, attributable to the transfer of electrons from the photoexcited sensitizing dye to the working electrode.
